# EUROPEAN PATENT SPECIFICATION

(11) **EP 1 558 764 B1**
(45) Date of publication and mention of the grant of the patent: **05.01.2011**
(21) Application number: 03781775.6
(22) Date of filing: 05.11.2003
(51) Int. Cl.: C12Q 1/68, C12P 19/34, C07H 21/04

(54) **UNIVERSAL TAG ASSAY**
UNIVERSELLER TAG-ASSAY
ESSAI DE MARQUEUR UNIVERSEL

(30) Priority: 06.11.2002 US 424656 P; 24.04.2003 US 424542
(43) Date of publication of application: 03.08.2005
(73) Proprietor: Geneohm Sciences, San Diego, CA 92121 (US)
(72) Inventor: CROTHERS, Donald, M., Northford, CT 06472 (US); HOLMLIN, R., Erik, San Diego, CA 92101 (US)
(74) Representative: Raynor, Simon Mark
(86) International application number: PCT/US2003/035378
(87) International publication number: WO 2004/044549

(56) References cited:
- WO-A-00/58516
- US-A- 5 605 662
- US-A- 5 854 033
- US-A- 5 981 297
- US-A1- 2001 021 534
- US-A1- 2001 026 919
- US-A1- 2002 001 801
- US-B1- 6 177 248
- US-B1- 6 180 346
- US-B1- 6 232 067
- US-B1- 6 352 828

## Description

### Field of the Invention

The present invention relates to a universal tag assay wherein tagged molecules having identifier tags corresponding to targets are incubated with a universal detector having detection probes, and hybridization of an identifier tag to a complementary detection probe indicates the presence of the corresponding target in the sample being assayed. In particular, the invention relates to detecting target nucleotide sequences in a sample using target-dependent processes to generate tagged molecules having identifier tags that correspond to target nucleotide sequence, wherein tagged molecules are incubated with a universal detector having detection probes and hybridization of identifier tags to complementary detection probes is measured. Preferred embodiments include use of the universal tag assay for detecting variant sequences including single nucleotide polymorphisms (SNPs), allelic variants, and splice variants. Preferred embodiments further include the use of ruthenium amperometry to detect hybridization of tagged DNA or RNA molecules to detection probes immobilized on a universal detector, preferably a universal chip having gold or carbon electrodes.

### Background of the Invention

Hybridization of polynucleotides to other polynucleotides having at least a portion of complementary nucleotide sequence by Watson-Crick base pairing is a fundamental process useful in a wide variety of research, medical, and industrial applications. Detecting the hybridization of a probe to a polynucleotide containing a target sequence is useful for gene expression analysis, DNA sequencing, and genomic analysis. Particular uses include identification of disease-related polynucleotides in diagnostic assays, screening for novel target polynucleotides in a sample, identification of specific target polynucleotides in mixtures of polynucleotides, identification of variant sequences, genotyping, amplification of specific target polynucleotides, and therapeutic blocking of inappropriately expressed genes, *e.g.* as described in Sambrook et al., Molecular Cloning: A Laboratory Manual, 2nd Edition (Cold Spring Harbor Laboratory, New York, 1989); Keller and Manak, DNA Probes, 2nd Edition (Stockton Press, New York, 1993); Milligan et al., 1993, J Med Chem, 36: 1923-1937; Drmanac et al., 1993. Science, 260: 1649-1652; Bains, 1993, J DNA Seq Map, 4: 143-150.

Immobilized probes are useful for detecting polynucleotides containing a target nucleotide sequence, where each immobilized probe is functionally connected to a support and the hybridization of a polynucleotide to the immobilized probe can be detected. Most commonly, DNA probes are used to detect polynucleotides containing a target nucleotide sequence complementary to the probe sequence. The support for immobilized probes may be a flat surface, often called a "chip," or the support may be the surface of a bead or other particle. Probes are usually immobilized in a known arrangement, or array, which provides a medium for matching known and unknown polynucleotides based on base-pairing rules. Preferably, the process of identifying the unknowns identified using a probe array is automated. Microarrays having a large of number of immobilized probes of known identity are used to determine complementary binding, allowing massively parallel studies of gene expression and gene discovery. For example, an experiment with a single DNA chip can provide researchers information on thousands of genes simultaneously. For example, Hashimoto *et al.* disclose an array of immobilized single-stranded probes wherein at least one probe has a nucleotide sequence complementary to the target gene(s) to be detected, such that each probe is immobilized onto the surface of an electrode or the tip of an optical fiber and an electrochemically or optically active substance capable of binding to double-stranded nucleic acid is used to detect hybridization of target genes to complementary immobilized probes (U.S. Pat. Nos. 5,776,672 and 5,972,692).

### Universal chips

Under some circumstances, a drawback to chip technology is that each chip must be manufactured specifically for the sequences to be detected, with a set of immobilized probes that are designed to be complementary to specific sequences to be detected. Chips specific for a single organism require a large manufacturing investment, and the chips can only be used for a narrowly defined range of samples. In contrast, a "universal chip" or "universal array" is organism-independent because the probes are not targeted to organism-specific sequences or products. Chips specific for a specific tissue, physiological condition, or developmental stage, often used for gene expression analysis, can likewise require a substantial manufacturing investment for use with a limited range of samples. A universal chip provides an unrestricted approach to studying tissues, physiological conditions, or developmental stages of interest. Manufacturing quality control can be improved by using a universal chip for polynucleotide detection.

One approach to universal chip design involves attaching a set of oligonucleotide probes to a chip surface, where the set of oligonucleotide probes includes all possible sequences of oligonucleotides that are 5, 6, 7, 8, 9, 10 or more nucleotides in length. The probes needed for these arrays can be designed using a simple combinatorial algorithm. The chip is incubated with a mixture that may contain DNA, cDNA, RNA or other hybridizable material, and hybridization to each probe of known sequence is measured. However, the specificity of such an array may be impaired because different sequences may have different requirements for stringent hybridization. In addition, such a universal array does not prevent false positives resulting from frameshifting where, for example in a universal array having probes that are six nucleotides long, the final four nucleotides of a sample polynucleotide may hybridize to the complementary final four nucleotides of a six-nucleotide probe, but the same sample polynucleotide would not hybridize to the entire six-nucleotide probe sequence.

Suyama et al. (2000, Curr Comp Mo/ Biol 7:12-13) disclose a universal chip system for gene expression profiling of a sample, where the chip system utilizes "DNA computing" instead of binding of transcripts to probes. The DNA computing system of Suyama *et al.* indirectly determines which transcripts are present by measuring binding of coded adapters to a universal set of immobilized probes on the universal chip. Only those coded adapters with a region complementary to a region of a transcript present in a sample will undergo the subsequent manipulations and the processing steps that generate adapters capable of binding to probes on the universal chip.

### Tags

An alternative approach to manufacturing a universal chip involves the using a set of tag sequences that do not naturally occur in the target polynucleotides, where the tags bind to complementary probes on a universal chip. Tags for such uses are sometimes known as "address tags" or "zip codes" or are considered to be analogous to "bar codes" for identifying targets. Detection, identification, tracking, sorting, retrieving or other manipulations are then directed at tag sequences and not the sequences of the target polynucleotides. Oligonucleotide tags may be covalently attached to or incorporated into polynucleotides. Tags may become associated with a polynucleotide by hybridization of a separate oligonucleotide which functions as a linker by virtue of having at least two domains, one with tag sequence complementary to a probe and one with sequence complementary to at least a portion of the target polynucleotide. Systems employing oligonucleotide tags have been proposed as means for manipulating and identifying individual molecules in complex mixtures, for example to detect polynucleotides having target nucleotide sequences, or as an aid to screening genomic, cDNA, or combinatorial libraries for drug candidates. Brenner and Lerner, 1992, Proc Natl Acad Sci, 89: 5381-5383; Alper, 1994, Science, 264: 1399-1401; Needels et al., 1993, Proc Nat Acad Sci, 90: 10700-10704.

### Spurious signals

The usefulness of tagged polynucleotides depends in large part on success in achieving specific hybridization between a tag and its complementary probe immobilized to a surface. For an oligonucleotide tag to successfully identify a polynucleotide, the number of false positive and false negative signals must be minimized. Unfortunately, spurious signals are not uncommon because base pairing and base stacking free energies can vary widely among nucleotide sequences in a duplex or triplex structure. For example, a tag-probe duplex having a different number of guanosine-cytosine (G-C) pairs than another duplex will have a different melting temperature, such that tag-probe duplexes with differing G-C ratios will have different stringency requirements for hybridization. In addition, a tag-probe duplex consisting of a repeated sequence of adenosine (A) and thymidine (T) bound to its complement may have less stability than a duplex of equal length consisting of a repeated sequence of G and C bound to a partially complementary target containing a mismatch, due to differences in stacking energy. Special reagents are often required to balance these differences in stacking energy.

Spurious signals can also result from "frameshifting" as described above. This problem has been addressed by employing a "comma-less" code, which ensures that a probe out of register (frameshifted) with respect to its complementary tag would result in a duplex with one or more mismatches for each of its codons, which forms an unstable duplex.

In view of the above problems with spurious signals, researchers have developed various oligonucleotide-based tagging systems which provide a sufficient repertoire of tags, but which also minimize the occurrence of false positive and false negative signals without the need to employ special reagents for altering natural base pairing and base stacking free energy differences, or elaborate encoding systems for comma-less codes. Such tagging systems find applications in many areas, including construction and use of combinatorial chemical libraries, large-scale mapping and sequencing of DNA, genetic identification, and medical diagnostics.

*Brenner et al.* disclose a 'universal' chip system that attaches tags to the ends of polynucleotide fragments through reactive moieties, where spurious signals are avoided by designing a repertoire of multi-subunit oligonucleotide tags with sequences such that the stability of any mismatched duplex or triplex between a tag and a complement to another tag is far lower than that of any perfectly matched duplex between the tag and its own complement. U.S. Pat. Nos. 5,604,097, 5,654,413, 5,846,719, 5,863,722, 6,140,489, 6,150,516, 6,172,214, 6,172,218, 6,352,828, 6,235,475. Morris *et al*. (U.S. Pat. No. 6,458,530, EP 0799897) disclose the use of tags and arrays of complementary probes to label and track compositions including cells and viruses, and to facilitate analysis of cell and viral phenotypes.

An alternate approach involves multicomponent tagging systems where tags are not attached to polynucleotides but rather, are found on separate components that are hybridized to the polynucleotides in order to adapt, index, and/or detect polynucleotides having a defined nucleotide sequence. The method disclosed in U.S. Pat. No. 6,261,782 and related patents and applications (Lizardi *et al*.) permits the user to sort and identify target polynucleotides in a sample by generating "sticky ends" using nucleic acid cleaving reagents, indexing the cleaved polynucleotide fragments into sets by adding adapter-indexer oligonucleotides with ends complementary to various sticky ends to the sample, adding ligator-detector oligonucleotides with sticky ends complementary to the sticky ends of adapter-indexers, hybridizing the entire sample with a plurality of detector probes, covalently coupling the ligator-detectors to the detector probes, and finally detecting coupling of ligator-detectors to the detector probes.

Another multicomponent system is disclosed by Balch et al., in U.S. Pat. No. 6,331,441, using a bifunctional linker with a domain that hybridizes to an immobilized capture probe in a universal array and a domain that hybridizes to an analyte containing a target. Balch et *al.* also discloses amplification of a target polynucleotide to generate amplicons containing both target sequence and a unique universal sequence complementary to a capture probe, where the unique universal sequence may be introduced through PCR or LCR primers (U.S. Pat. No. 6,331,441).

US 2001/026919 A describes hybridization assays, as well as kits, primers and arrays for use in the assays. A population of tagged target nucleic acids generated from a population of tagged gene specific primers is contacted with an array of tag complements under hybridization conditions and the presence of any resultant hybridized tag target nucleic acid-tag complement structures is detected. The arrays may be used in a number of different applications, e.g. differential gene expression analysis.

US 6352828 describes a method of tracking, identifying, and/or sorting classes or subpopulations of molecules by the use of oligonucleotide tags selected from a minimally cross-hybridizing set. The tags are used for sorting polynucleotides by hybridizing tags attached to the polynucleotides to their complements on solid phase supports. This provides an automated system for manipulating and sorting polynucleotides, which is useful in large-scale DNA sequencing, where many target polynucleotides or segments of a single target polynucleotide are sequenced simultaneously.

US 6180346 describes a method of preparing an electrode by electropolymerizing a film on the conductive working surface of an electrode. The film is formed from a co-polymer of a mediator and a functionalized moiety having a carboxylate group. A DNA probe is attached covalently to the carboxylate group via a carbodiimide reaction followed by amidation of an amino-linked single-stranded DNA.

### Summary of the Invention

The present invention provides
A method for detecting a target nucleotide sequence in a sample, said method comprising the steps of:
a) generating at least one tagged molecule comprising at least one identifier tag selected as an identifier for said target nucleotide sequence, wherein said identifier tag is generated only when said target nucleotide sequence corresponding to said identifier tag is present in said sample;
b) incubating said at least one tagged molecule with a universal detector having at least one detection probe complementary to said identifier tag, said universal detector comprising an array of detection probes coupled to electrochemical detection means, said array comprising electrodes attached to a substrate; and
c) detecting hybridization of any said identifier tag to any said detection probe complementary to said identifier tag, wherein said hybridization of any said identifier tag to any said detection probe complementary to said identifier tag indicates said target nucleotide sequence corresponding to said identifier tag is present in said sample.

The present invention also provides
A composition for detecting targets in a sample using a universal tag assay comprising:
a) a set of minimally cross-hybridizing tags and probes selected such that at least one tag will serve as an identifier tag for a target in a sample being assayed and each said identifier tag has at least one complementary detection probe in said set;
b) at least one tagged molecule comprising at least one said identifier tag, wherein said identifier tag is generated only when said target corresponding to said identifier tag is present in said sample being assayed; and
c) a universal detector comprising at least one said detection probe coupled to an electrochemical detection means such that hybridization of said any identifier tag to any said complementary detection probe can be measured
wherein said universal detector comprises an array of detection probes coupled to detection means, said array comprising electrodes attached to a substrate;
wherein measuring hybridization of any said identifier tag to any said complementary detection probe indicates that said target corresponding to said identifier tag is present in said sample being assayed.

The present disclosure provides methods and compositions for detecting targets in a sample using a universal tag assay. The universal tag assay disclosed and claimed herein provides tags and probes and universal detectors for use in a universal tag assay that advantageously minimizes spurious signals without the need to employ special conditions or special reagents. Targets are detected using the universal tag assay of the present invention by generating tagged molecules having identifier tags corresponding to targets, incubating tagged molecules with a universal detector having detection probes, and measuring hybridization of identifier tags to complementary detection probes, where hybridization of an identifier tag to its complementary detection probe indicates the presence of the target corresponding to that identifier tag.

The universal tag assay disclosed herein includes but is not limited to: a) a set of minimally cross-hybridizing tags and probes selected such that at least one tag will serve as an identifier tag for each target being assayed and each tag has a complementary detection probe in the universal detector; b) tagged molecules generated from the sample being assayed, where a tagged molecule containing an identifier tag for a target is only generated when that target is present in the sample; and c) a universal detector having detection probes coupled to a detection means in a manner such that hybridization of tags to complementary detection probes on the universal detector can be detected. The universal tag assay disclosed herein provides that detecting hybridization of an identifier tag to its complementary detection probe indicates the presence of the corresponding target in the sample being assayed. A tagged molecule may be a tagged target that contains a copy or complement of a target and the identifier tag for that target. Alternately, a tagged molecule may contain an identifier tag molecule for a target and no copy or complement of the target. Tagged molecules may be generated using target-dependent amplification methods, or may be generated by target-dependent methods that do not employ amplification of target, or by a combination of methods.

In accordance with certain methods described herein, the presence of a target nucleotide sequence in a sample may be detected by generating at least one tagged molecule in response to the target nucleotide sequence in a target-dependent manner, incubating tagged molecules with a universal detector having at least one detection probe, and measuring hybridization of identifier tags to complementary detection probes. A tagged molecule may be a tagged target that contains a copy or complement of the target nucleotide sequence and an identifier tag for that target nucleotide sequence. Alternately, a tagged molecule contains an identifier tag for a particular target sequence and no copy or complement of the target nucleotide sequence. Tagged molecules are incubated with a universal detector having detection probes coupled to a detection means, and hybridization of an identifier tag to a complementary detection probe on the universal detector generates a signal that indicates the presence of the corresponding target nucleotide sequence in the sample.

The universal detector includes detection probes attached to a surface, film, or particle, wherein detection probes are coupled to a detection means such that hybridization of a tag to a complementary probe can be detected. Detection probes may be immobilized in a fixed array, or may be attached to a surface, film, or particle in a manner that permits changing the location of the detection probes. One or more detection probes may be coupled to a particle such as a bead. Detection probes may be attached to a surface, film, or particle by covalent bonds, ionic bonds, or electrostatic interactions, and the attachment may be reversible. Alternately detection probe may be coupled to a detection means in solution, such that hybridization of a tag to a complementary probe can be detected.

Preferably, the universal tag assay of the present disclosure provides a universal chip that includes detection probes immobilized to a support including a detection means, such that hybridization of tags to complementary detection probes can be measured. Detection means for measuring hybridization of tags to complementary detection probes can be electrochemical, fluorescent, colorimetric, radiometric or magnetic. In particular, the universal tag assay of the present invention provides an array of detection probes coupled to electrodes attached to a substrate, and hybridization of oligonucleotide tags to oligonucleotide detection probes is detected by electrochemical methods. More preferably, gold or carbon electrodes are used to detect tag binding to detection probes. Even more preferably, hybridization of identifier tags to detection probes immobilized to gold or carbon electrodes is detected by ruthenium amperometry. The electrodes may be coated with avidin which can be bound by biotin-labelled oligonucleotides. Alternately, electrodes may be coated with another protein which can be bound by oligonucleotides derivatized with a moiety that binds the protein coating the electrode.

One aspect of the disclosure provides a method for detecting a target nucleotide sequence in a sample, where the method may include but is not limited to the following steps: a) obtaining template containing the target nucleotide sequence; b) amplifying the template to generate at least one tagged molecule having at least one copy or complement of the target nucleotide sequence and at least one tag sequence chosen as an identifier tag for the target nucleotide sequence; d) incubating at least one tagged molecule with a universal detector having detection probes coupled to a detection means; e) detecting hybridization of identifier tags to complementary detection probes on a universal detector. In accordance with the universal tag assay disclosed herein, detecting hybridization of an identifier tag to a complementary detection probe on a universal detector indicates the presence of the corresponding target nucleotide sequence in the sample being assayed. Another aspect provides a method for detecting multiple target nucleotide sequences in a sample, wherein each target nucleotide sequence has a distinct identifier tag.

One preferred embodiment of the disclosure provides a method for detecting a target nucleotide sequence in a sample, where the method may include but is not limited to the following steps: a) obtaining template containing the target nucleotide sequence; b) amplifying the template to generate at least one tagged molecule having at least one copy or complement of the target nucleotide sequence and at least one tag sequence chosen as an identifier tag for the target nucleotide sequence, as well as optional additional exogenous nucleotide sequences including sequences involved in trimming amplification products; c) trimming amplification products to generate at least one tagged molecule containing tag sequence chosen as an identifier tag for the target nucleotide sequence; d) incubating at least one tagged molecule with a universal detector capable of finding tag sequence; and e) detecting hybridization of identifier tags to complementary detection probes on a universal detector. In accordance with the universal tag assay disclosed herein, detecting hybridization of an identifier tag to a complementary detection probe on a universal detector indicates the presence of the corresponding target nucleotide sequence in the sample being assayed. Another aspect provides a method for detecting multiple target nucleotide sequences in a sample, wherein each target nucleotide sequence has a distinct identifier tag.

Another preferred embodiment of the disclosure provides a method for detecting a target nucleotide sequence in a sample, where the method may include but is not limited to the following steps: a) obtaining template containing the target nucleotide sequence; b) amplifying the template to generate at least one tagged molecule having at least one tag sequence chosen as an identifier tag for the target nucleotide sequence and optionally, at least one copy or complement of the target nucleotide sequence, as well as additional exogenous nucleotide sequences including sequences involved in trimming amplification products; c) trimming amplification products to generate at least one tagged molecule containing the identifier tag for the target nucleotide sequence and no copy or complement of the target nucleotide sequence; d) incubating at least one tagged molecule with a universal detector having a set of detection probes coupled to a detection means; e) detecting hybridization of identifier tags to complementary detection probes on a universal detector. In accordance with the universal tag assay disclosed herein, detecting hybridization of an identifier tag to a complementary detection probe on a universal detector indicates the presence of the corresponding target nucleotide sequence in the sample. Another aspect provides a method for detecting multiple target nucleotide sequences in a sample, wherein each target nucleotide sequence has a distinct identifier tag.

Yet another preferred object of the present disclosure provides a set of complementary tags and probes suitable for use with the universal tag assay disclosed herein, preferably a set of minimally cross-hybridizing oligonucleotides wherein all tag-probe duplexes have the same or similar melting temperature and stacking energy. Another object provides a set of probes and a set of tags such that each tag in the set has a complementary detection probe coupled to a detection means in the universal detector, with the result that not only does hybridization of a tag to its complementary detection probe reliably indicate the presence of the corresponding target in a sample, but also the absence of hybridization of a tag to its complementary detection probe reliably indicates the absence of the corresponding target in a sample. Preferably, the reliability of universal tag assay is increased by including tags and probes that serve as internal controls for reagent quality, hybridization conditions, and other parameters.

In accordance with one preferred aspect of the methods disclosed for detecting target nucleotide sequences disclosed herein, rolling circle (RC) amplification of a suitable template may be used for the amplification step. In embodiments using RC amplification, the RC probe used to amplify template containing a target nucleotide sequence includes a portion of sequence complementary to the target nucleotide sequence and further includes sequence complementary to an identifier tag for that target nucleotide sequence, such that the amplification products contain a copy of the target nucleotide sequence and a distinct identifier tag sequence capable of hybridizing to a detection probe. Preferably, products of RC amplification contain additional exogenous sequences not found in the target nucleotide sequence or tag sequence, which may include but are not limited to sequences involved in trimming amplification products, sequences involved in primer binding, or sequences involved in forming polymerase promoters. Alternately, RC amplification may be carried out on copies or complements of nucleotide sequence. Preferred embodiments provide that RC amplification can be carried out in linear mode or non-linear mode. In one preferred embodiment, RC amplification in linear mode generates single-stranded amplification products. In another preferred embodiment, RC amplification in non-linear, or exponential mode using at least one additional primer complementary to a portion of the amplification product generates double-stranded amplification products, preferably hyperbranched amplification products. The template for RC amplification may be DNA or RNA, including but not limited to genomic DNA, cDNA, PCR products, ligation products including LCR products, RC amplification products, synthetic DNA, mRNA, rRNA, RC transcription products, or synthetic RNA. Single-stranded template may be obtained by denaturing double-stranded DNA, preferably to generate single-stranded template from the target strand containing at least one target nucleotide sequence. The double-stranded DNA may be genomic DNA, cDNA, PCR products, or ligation products including LCR products.

In accordance with another preferred embodiment of the disclosure, RC amplification is used to amplify PCR products or LCR products containing a copy or complement of the target nucleotide sequence. In one embodiment, a PCR product containing a complement of the target nucleotide sequence is amplified using an RC probe having a copy of the target nucleotide sequence and sequence complementary to an identifier tag for that target nucleotide sequence, such that the amplification products contain at least one complement of the target nucleotide sequence and an identifier tag capable of hybridizing to a detection probe. Optionally, the RC amplification products are trimmed.

In accordance with another preferred embodiment of the disclosure, more than one amplification of target nucleotide sequence is carried out. Ligase chain reaction (LCR), non-enzymatic ligation, or PCR can be used to amplify target nucleotide sequence. LCR products, non-enzymatic ligation products, and PCR products may be amplified in a subsequent amplification step, preferably an RC amplification step.

LCR or non-enzymatic ligation amplification of target nucleotide sequence includes but is not limited to the following steps: a) if necessary, obtaining single-stranded template having at least one target nucleotide sequence; b) contacting the template with a plurality of oligonucleotide ligation primers, where at least one pair of the ligation primers is designed to hybridize to at least one target nucleotide sequence on the template, such that the 5' end of one of the pair of ligation primers hybridizes adjacent to the 3' end of the other of the pair of ligation primers; c) incubating template and ligation primers under conditions that promote adjacent hybridization of at least one pair of ligation primers to the target nucleotide sequence on the template and ligation of any adjacent hybridized pair of ligation primers to form at least one ligation product that includes sequence complementary to the target nucleotide sequence; d) dissociating the ligation product from the template; e) repeating the hybridization and ligation steps as desired; f) recovering the ligation products for use in subsequent amplification steps. In one preferred embodiment, of the disclosure ligation reactions, preferably LCR, are repeated using temperature cycling for exponential amplification of the target nucleotide sequence.

In a preferred embodiment, of the disclosure each ligation primer including sequence complementary to the target nucleotide sequence on the target strand also includes exogenous nucleotide sequence complementary to a portion of the "backbone" of a circularizable RC padlock probe in linear form that contains a copy of the target nucleotide sequence and a complement of an identifier tag sequence. In such an embodiment, the RC padlock probe in linear form has 3' sequence corresponding to a region of target nucleotide sequence, and 5' sequence corresponding to a region of target nucleotide sequence, where the 3' and 5' sequence is separated by a "backbone" region that does not contain sequence corresponding to target nucleotide sequence. The ligation product includes 5' and 3' exogenous nucleotide sequence complementary to a portion of the backbone of the RC padlock probe, where the sequence complementary to a portion of the backbone of the RC padlock probe flanks sequence complementary to the target nucleotide sequence. The ligation product is then incubated with at least one RC padlock probe in linear form under conditions that promote hybridization of the RC padlock probe to the ligation product, such that the 5' end of the RC padlock probe is adjacent to the 3' end of the RC padlock probe and the 5' and 3' ends are ligated to form a circularized RC padlock probe. DNA polymerase is added to the complex formed by the RC padlock probe and the ligation product, under conditions that permit RC amplification of the RC padlock probe using the ligation product as a polymerization primer. In this embodiment, the amplification product is a single-stranded DNA molecule containing multiple copies of the RC probe sequence, including sequence complementary to the target nucleotide sequence and identifier tag sequence corresponding to the target nucleotide sequence. This amplification product may be used as a tagged molecule in the universal tag assay. Optionally, the amplification product may contain additional exogenous nucleotide sequence. The amplification product ma include modified nucleotides, addressable ligands, or other modifications. In another prefered of the disclosure embodiment, the amplification product includes at least one additional exogenous nucleotide sequence involved in post-amplification trimming of the amplification product to yield smaller tagged molecules for use in the universal tag assay. The amplification product may also contain primer binding sites for additional amplification steps, for example to generate double-stranded amplification products. The amplification product may further include sequences involved in forming promoter regions, preferably for polymerases.

In accordance with another preferred embodiment of the disclosure additional exogenous nucleotide sequences not found in the target or the identifier tag may be introduced during an amplification step, wherein such exogenous nucleotide sequences may include sequences involved in trimming amplification products. In one embodiment, the exogenous nucleotide sequence may contain self-complementary sequences that form hairpin structures. These self-complementary sequences that form hairpin structures may contain at least one restriction enzyme recognition site for a restriction enzyme involved in the trimming step, and suitable restriction enzymes include Type II restriction enzymes such as EcoRI, or Type IIS restriction enzymes such as FokI. In another embodiment, exogenous nucleotide sequences introduced during an amplification step encode one strand of the restriction enzyme recognition site, and a double-stranded restriction enzyme recognition site is formed upon addition of at least one auxiliary oligonucleotide. Suitable restriction enzymes include Type II restriction enzymes such as *EcoRI,* or Type IIS restriction enzymes such as *FokI.*

In accordance with another preferred embodiment of the disclosure, additional exogenous nucleotide sequence not found in the target nucleotide sequence of the tag sequence may be introduced during an amplification step, wherein such exogenous sequences may include sequences involved in forming promoter regions for binding of polymerases to amplification products. In a preferred embodiment, double-stranded amplification product includes exogenous nucleotide sequence encoding a promoter for DNA or RNA polymerase, preferably T7 RNA polymerase, T7 DNA polymerase, *Bst* DNA polymerase, or phi 29 (φ29) DNA polymerase.

In accordance with one preferred embodiment disclosed, ligation reactions are used to identify variant or polymorphic sequences of the target nucleotide sequence present in a sample. The variant sequence may be a single nucleotide polymorphism (SNP). Alternately, the variant sequence represents mutant or allelic forms, or splice variants, of a target nucleotide sequence. In a preferred embodiment, the amplification step is carried out using a plurality of RC padlock probes in linear form having sequences complementary to variant sequences of the target nucleotide sequence, wherein each RC probe in linear form is complementary to a single variant sequence and each probe includes complement of the identifier tag for that variant sequence. A sample is incubated with this plurality of RC padlock probes in linear form under conditions suitable for hybridization and ligation of RC padlock probes, such that only those RC padlock probes complementary to the variant sequence present in the sample will hybridize to the variant sequence and be ligated to form a circularized RC padlock probe suitable to generate tagged molecules for use with the universal tag assay. Hybridization of an identifier tag to a detector probe indicates which variant sequence was present, because only the RC probe complementary to the variant sequence present in the sample was circularized and amplified to generate the identifier tag capable of binding to a detection probe.

In accordance with another preferred embodiment of the disclosure, a plurality of variant sequences of the same or different target nucleotide sequences can be detected in a single reaction using a plurality of RC padlock probes in linear form as described above, wherein each RC padlock probe in linear form includes sequence complementary to a single variant sequence and complement of the identifier tag for that variant sequence. Hybridization of identifier tags to complementary detection probes indicates which variant sequences are present in the sample being assayed, because only those RC probes complementary to the variant sequences present in the sample were circularized and amplified to generate tagged molecules containing identifier tags capable of binding to complementary detection probes.

Alternately, ligation of primers can be used to identify variant or polymorphic sequences present in a sample. Ligation may be carried out using LCR or non-enzymatic ligation. A sample is incubated with ligation primers having sequences complementary to variant sequences of the target nucleotide sequence under conditions suitable for hybridization and ligation, wherein only those ligation primers complementary to the variant sequence present in the target strand template will hybridize to the template and form at least one ligation product having sequence that is complementary to the variant target nucleotide sequence present in the template. In another preferred embodiment, the set of ligation primers includes primers having exogenous sequence such that any ligation product includes exogenous nucleotide sequence flanldng (3' and 5') sequence complementary to a portion of the variant target nucleotide sequence. A plurality of variant sequences of the same or different target nucleotide sequences may be detected in a single reaction using a plurality of ligation primers as described above, wherein only those ligation primers having sequence complementary to a variant sequence will produce a ligation product complementary to that variant sequence. Ligation products having sequence complementary to variant sequences may be amplified to generate tagged molecules suitable for use with the universal tag assay disclosed herein. Optionally, ligation reactions can be carried out on PCR products containing copies or complements of target nucleotide sequence.

In another preferred embodiment of the disclosure ligation primers complementary to variant sequences further contain exogenous sequence. In one embodiment, the 5' end of the primer complementary to the region of target sequence 3' ("downstream") to the point of variant sequence contains identifier tag sequence, and the 3' end of the primer complementary to the region of target sequence 5' ("upstream") to the point of variant sequence contains an RNA polymerase promoter sequence. The two primers are called the tag sequence primer and the promoter sequence primer, respectively. A ligation product is formed by ligation of a pair of primers complementary to the variant sequence present in the sample being assayed. The ligation product has an identifier tag, sequence complementary to the variant sequence, and one strand of an RNA polymerase promoter. Upon addition of an auxiliary oligonucleotide complementary to the promoter sequence, RNA transcription can be initiated. Transcription of the tag sequence occurs only if ligation resulted in joining the two halves of the target sequence. The target can be genomic DNA, RNA, or a copy of the target amplified by methods such as PCR, LCR, or RC amplification. In a preferred embodiment, the strand complementary to target is removed, for example by biotin or hybridization capture or by selective exonuclease digestion, to enhance the efficiency of ligation of the promoter ligation primer and tag ligation primer. A plurality of variant sequences of the same or different target nucleotide sequences may be detected in a single reaction using a plurality of ligation primers as described above, wherein only those ligation primers having sequence complementary to a variant sequence will generate a ligation product complementary to that variant sequence. Transcription of ligation products generates tagged RNA molecules containing identifier tags. Hybridization of RNA identifier tags to complementary detection probes indicates which variant sequences are present in the sample being assayed. In an alternative embodiment, the promoter-tag ligation can result from an LCR amplification, wherein the LCR primer complementary to the target sequence in the tag ligation primer does not contain the complement of the tag sequence.

Another preferred embodiment of the disclosure is directed to methods for identifying an organism or individual by detecting a target nucleotide sequence chosen to serve as a distinguishing feature. An organism or individual is identified using some or all of the following steps: a) obtaining a sample from the organism or individual, where the sample contains template having at least one target nucleotide sequence; b) generating tagged molecules in a target-dependent manner; c) optionally, trimming tagged molecules to generate smaller tagged molecules; d) incubating tagged molecules with a universal detector having an array of detection probes coupled to a detection means; and e) detecting hybridization of identifier tags to complementary detection probes. In one embodiment, an organism or individual is identified by hybridization of an identifier tag to its complementary detection probe, where the tagged molecules containing an identifier tag corresponding the target were generated because the target was present in the sample being assayed. In another embodiment, an organism or individual may be identified not only by hybridization of an identifier tag to its complementary detection probe, which reliably indicates the presence of the corresponding target in the sample being assayed, but also by the absence of hybridization of an identifier tag to its complementary detection probe, which reliably indicates the absence of the corresponding target in the sample being assayed. Preferably, at least one internal control is included in each assay in order to increase the reliability of results based on hybridization or lack of hybridization. More preferably, a plurality of internal controls are included. A plurality of targets in an individual or organism may be assayed using the universal tag assay. A plurality of individuals or organisms may be identified using the universal tag assay.

Some aspects of the present disclosure are described in the following numbered Paragraphs.

Paragraph 1: One aspect of the present invention is amethod for detecting a target nucleotide sequence in a sample, comprising:

a) generating at least one tagged molecule comprising at least one identifier tag selected as an identifier for said target nucleotide sequence, wherein said identifier tag is generated only when said target nucleotide sequence corresponding to said identifier tag is present in said sample;

b) incubating said at least one tagged molecule with a universal detector having at least one detection probe complementary to said identifier tag; and

c) measuring hybridization of any said identifier tag to any said detection probe complementary to said identifier tag;

wherein said hybridization of any said identifier tag to any said detection probe complementary to said identifier tag indicates said target nucleotide sequence corresponding to said identifier tag is present in said sample.

In some versions of the method of Paragraph 1, the at least one tagged molecule further comprises a copy or complement of said target nucleotide sequence.

In some versions of the method of Paragraph 1, the universal detector comprises detection probes coupled to a detection means for said measuring hybridization of any said identifier tag to any said detection probe complementary to said identifier tag. For example, the detection means may be electrochemical, fluorescent, colorimetric, radiometric or magnetic. In some embodiments, the detection means is electrochemical. In some embodiments, said detection probes coupled to a detection means are attached to a surface, film, or particle. For example, in some embodiments, said detection probes are attached to said surface, film, or particle by covalent bonds, ionic bonds, electrostatic interactions, or adsorption. In some embodiments, the detection probes are attached to a particle such as a bead. In some embodiments, the detection probes are attached to a plurality of particles. In some embodiments, the universal detector comprises an array of detection probes coupled to detection means, said array comprising electrodes attached to a substrate. In some embodiments, the electrodes are gold or carbon. In some embodiments, the electrodes are gold. In some embodimetns, the method further comprises measuring hybridization of any said identifier tag to any said detection probe complementary to said identifier tag by ruthenium amperometry. In some embodiments, the electrodes are coated with protein which can be bound by oligonucleotides derivatized with a moiety that binds said protein that coats said electrode. For example, in some embodiments, the electrodes are coated with avidin such that said electrodes can be bound by biotin-labelled oligonucleotides.

Paragraph 2: One preferred embodiment of the present disclosure is a method for detecting a target nucleotide sequence in a sample, comprising:

a) obtaining template comprising said target nucleotide sequence or complement thereof;

b) amplifying said template to generate at least one tagged molecule comprising at least one identifier tag selected as an identifier for said target nucleotide sequence;

c) incubating said at least one tagged molecule with a universal detector comprising detection probes coupled to a detection means, said detection probes comprising at least one detection probe complementary to said identifier tag; and

d) detecting hybridization of any said identifier tag to any said detection probe complementary to said identifier tag;

wherein hybridization of any said identifier tag to any said complementary detection probe indicates the presence of the corresponding target nucleotide sequence in the sample being assayed.

In one embodiment of the method of Paragraph 2, each target nucleotide sequence in said plurality of target nucleotide sequences has a distinct identifier tag, such that hybridization of each said distinct identifier tag to a complementary detection probe indicates the presence of the corresponding target nucleotide sequence.

In one embodiment of the method of Paragraph 2, said at least one tagged molecule comprises exogenous nucleotide sequence not found in said identifier tag or said target nucleotide sequence. In some embodiments, said exogenous sequence is sequence involved in trimming tagged molecules and said method further comprises trimming said at least one tagged molecule to generate at least one smaller tagged molecule.

In one embodiment of the method of Paragraph 2, said amplifying said template comprises rolling circle (RC) amplification comprising the steps of:

a) providing a rolling circle (RC) probe comprising sequence complementary to said template comprising target nucleotide sequence of complement thereof, and further comprising sequence complementary to said identifier tag selected as an identifier for said target nucleotide sequence;

b) incubating said RC probe with said template under conditions such that said RC probe will hybridize to complementary sequence on said template;

c) providing polymerase enzyme under conditions such that said polymerase replicates said RC probe, generating at least one amplification product comprising a tagged molecule having repeating copies of said identifier tag and said target nucleotide sequence or complement thereof;

d) incubating said amplification product with a universal detector comprising detection probes coupled to a detection means, said detection probes comprising at least one detection probe complementary to said identifier tag; and

e) detecting hybridization of any said identifier tag in said amplification product to any said detection probe complementary to said identifier tag;

wherein hybridization of any said identifier tag to any said complementary detection probe indicates the presence of the corresponding target nucleotide sequence in the sample being assayed. In some embodiments, said RC probe provided in Step a) is a circular RC probe. In some embodiment, the RC probe provided in Step a) is linear, such that Step b) further comprises incubating said RC probe with said template under conditions such that the 3' end of said RC probe and the 5' end of said RC probe will hybridize adjacently to contiguous complementary sequence on said template, and said 3' end and said 5' end are ligated to form a circular RC padlock probe. In some embodiments, said template is DNA or RNA. In some embodiments, said at least one amplification product further comprises exogenous sequence not found in said identifier tag or said template comprising target nucleotide sequence or complement thereof. For example, in some embodiments, said exogenous sequence is involved in primer binding to said amplification product, forming polymerase promoters on said amplification product, or trimming said amplification product. In some embodiments, said exogenous sequence involved in trimming said amplification product comprises self-complementary sequences that form hairpin structures. For example, in some embodiments, said self-complementary sequences that form hairpin structures comprise at least one restriction enzyme recognition site for a restriction enzyme involved in said trimming. In some embodiments, said at least one exogenous nucleotide sequence comprises sequences that form at least one restriction enzyme recognition site for a restriction enzyme involved in said trimming upon addition of at least one auxiliary oligonucleotide.

In some embodiments of the method of Paragraph 2, said amplifying said template comprises ligation-mediated amplification comprising:

a) providing at least one pair of ligation pimers comprising a first ligation primer having a 3' portion of sequence complementary to a portion of said template comprising said target nucleotide sequence or complement thereof, and a second ligation primer having a 5' portion of sequence complementary to a contiguous portion of said template comprising target nucleotide sequence or complement thereof, wherein at least one ligation primer further comprises an identifier tag selected as an identifier for said target nucleotide sequence;

b) incubating said at least one pair of ligation primers with said template under conditions such that said first and second ligation primers will hybridize to said template, said 3' end of said first ligation primer hybridizing adj acently to said 5' end of said second ligation primer;

c) ligating said 3' end of said first ligation primer to said 5' end of said second ligation primer, thereby generating a ligation product comprising a tagged molecule, wherein said tagged molecule comprises a copy or complement of said target nucleotide sequence and further comprises at least one identifier tag corresponding to said target nucleotide sequence;

d) contacting said ligation product with a universal detector comprising detection probes coupled to a detection means, said detection probes comprising at least one detection probe complementary to said identifier tag; and

e) detecting hybridization of any said identifier tag in said ligation product to any said detection probe complementary to said identifier tag;

wherein hybridization of any said identifier tag to any said complementary detection probe indicates the presence of the corresponding target nucleotide sequence in the sample being assayed. In some embodiments, said template comprises target nucleotide sequence, such that said first ligation primer comprises sequence complementary to a portion of said target nucleotide sequence and said second ligation primer comprises sequence complementary a contiguous portion of said target nucleotide sequence, with the result that said first ligation primer and said second ligation primer hybridize to contiguous portions of template and are ligated, generating a ligation product comprising an identifier tag and sequence complementary to said target nucleotide sequence. In some embodiments, said template comprises complement of target nucleotide sequence, such that said first ligation primer comprises a portion of said target nucleotide sequence and said second ligation primer comprises a contiguous portion of said target nucleotide sequence, with the result that said first ligation primer and said second ligation primer hybridize to contiguous portions of template and are ligated, generating a ligation product comprising an identifier tag and said target nucleotide sequence. In some embodiments, said ligating said 3' end of said first ligation primer to said 5' end of said second ligation primer is carried out by enzymatic means.. In some embodiments, said enzymatic means is ligase. In some embodiments, said ligating said 3' end of said first ligation primer to said 5' end of said second ligation primer is carried out by non-enzymatic means. In some embodiments, said incubating and ligating steps are repeated using temperature cycling for amplification of said target nucleotide sequence. In some embodiments, the method comprises providing a plurality of pairs of ligation primers wherein at least one said pair of ligation primers of said plurality of pairs is complementary to each target nucleotide sequence of said plurality of target nucleotide sequences, and further wherein at least one ligation primer of each said pair comprises an identifier tag selected to serve as an identifier for said target nucleotide sequence. In some embodiments, the method comprises detecting at least one variant sequence of said target nucleotide sequence. For example, in some embodiments, said at least one variant sequence is a single nucleotide polymorphism (SNP), an allelic variant, or a splice variant. In some embodiments, said at least one variant sequence is a SNP.

Paragraph 3: Another preferred embodiment of the present disclosure is a method for detecting a target nucleotide sequence in a sample, comprising:

a) obtaining template comprising said target nucleotide sequence or complement thereof;

b) providing at least one pair of ligation primers comprising a first ligation primer having a 3' portion of sequence complementary to a portion of said template and a second ligation primer having a 5' portion of sequence complementary to a contiguous portion of said template, wherein each ligation primer of said pair of ligation primers further comprises exogenous sequence not complementary to said template;

c) incubating said at least one pair of ligation primers with said template under conditions such that said first and second ligation primers will hybridize to said template, said 3' end of said first ligation primer hybridizing adjacently to said 5' end of said second ligation primer;

d) ligating said 3' end of said first ligation primer to said 5' end of said second ligation primer, thereby generating a ligation product comprising a copy or complement of said target nucleotide sequence and further comprising exogenous 3' and 5' sequence not complementary to said template;

e) providing a rolling circle (RC) probe comprising sequence complementary to said ligation product and further comprising sequence complementary to an identifier tag selected to serve as an identifier for said target nucleotide sequence, wherein said sequence complementary to said ligation product comprises sequence complementary to said target nucleotide sequence or complement thereof, flanked by sequence complementary to said exogenous sequence of said ligation product;

f) incubating said RC probe with said ligation product under conditions such that said RC probe will hybridize to said ligation product;

g) providing polymerase enzyme under conditions such that said polymerase replicates said RC probe, generating at least one amplification product comprising a tagged molecule, wherein said tagged molecule comprises repeating copies of said identifier tag and repeating copies of said target nucleotide sequence or complement thereof;

h) incubating each said amplification product with a universal detector comprising detection probes coupled to a detection means, said detection probes comprising at least one detection probe complementary to said identifier tag; and

i) detecting hybridization of any said identifier tag in said amplification product to any said detection probe complementary to said identifier tag;

wherein hybridization of any said identifier tag to any said complementary detection probe indicates the presence of the corresponding target nucleotide sequence in the sample being assayed.

In some embodiments of the method of Paragraph 3, said RC probe provided in Step e) is a circular RC probe.

In some embodiments of the method of Paragraph 3, said RC probe provided in Step e) is linear, such that Step f) further comprises incubating said RC probe with said ligation product comprising said target nucleotide sequence or complement thereof, under conditions such that the 3' end of said RC probe and the 5' end of said RC probe will hybridize adjacently to contiguous complementary sequence on said template, such that said 3' end and said 5' end of said linear RC probe are ligated to form a circular RC padlock probe.

In some embodiments of the method of Paragraph 3, said at least one amplification product further comprises exogenous sequence not found in said ligation product. For example, in some embodiments, said exogenous sequence is involved in primer binding to said amplification product, forming polymerase promoters on said amplification product, or trimming said amplification product. In some embodiments, said exogenous sequence involved in trimming said amplification products comprises self-complementary sequences that form hairpin structures. In some embodiments, said self-complementary sequences that form hairpin structures comprise at least one restriction enzyme recognition site for a restriction enzyme involved in said trimming step. In some embodiments, said at least one exogenous nucleotide sequence comprises sequences that form at least one restriction enzyme recognition site for a restriction enzyme involved in said trimming step upon addition of at least one auxiliary oligonucleotide.

Paragraph 4: One aspect of the present disclosure is a universal tag assay comprising:

a) a set of minimally cross-hybridizing tags and probes selected such that at least one tag will serve as an identifier tag for a target in a sample being assayed and each said identifier tag has at least one complementary detection probe in said set;

b) at least one tagged molecule comprising at least one said identifier tag, wherein said identifier tag is generated only when said target corresponding to said identifier tag is present in said sample being assayed; and

c) a universal detector comprising at least one said detection probe coupled to a detection means such that hybridization of said any identifier tag to any said complementary detection probe can be measured;

wherein measuring hybridization of any said identifier tag to any said complementary detection probe indicates that said target corresponding to said identifier tag is present in said sample being assayed.

In one embodiment of the method of Paragraph 4, said at least one tagged molecule comprises the identifier tag for a target and further comprises a copy or complement of said target.

In one embodiment of the method of Paragraph 4, said at least one tagged molecule comprises the identifier tag molecule for a target and does not contain a copy or complement of said target.

Paragraph 5: Another preferred embodiment of the present disclosure is a set of complementary tags and probes suitable for use with the universal tag assay of Claim 48, wherein said set comprises minimally cross-hybridizing oligonucleotides wherein all duplexes formed by said complementary tags and probes have approximately the same melting temperature and stacking energy.

### Brief Description of the Drawings

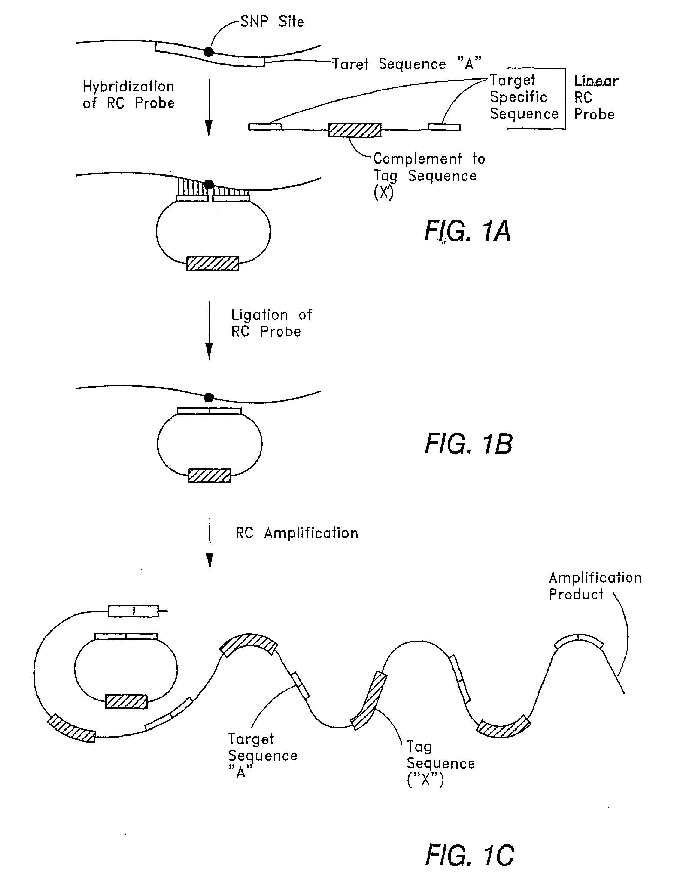

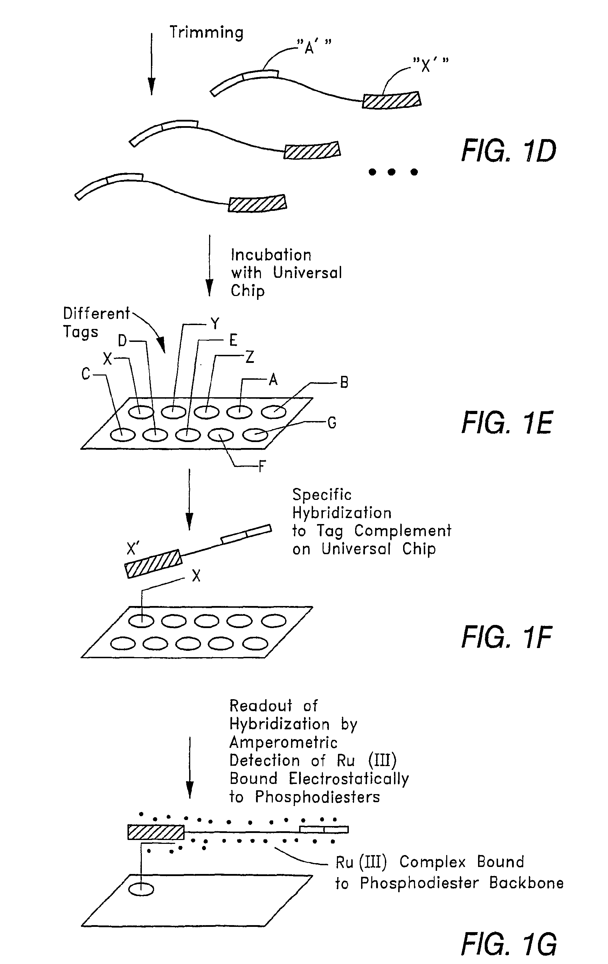

**Figure 1****. A.** Hybridizing a rolling circle (RC) padlock probe in linear form to target nucleotide sequence "A" on template. **B.** Ligating 3' and 5' ends of RC probe to form circularized RC padlock probe including target nucleotide sequence A and complement of identifier tag sequence X. **C**. Amplification of the RC probe by RC amplification generates a single-stranded amplification product with repeating copies of target nucleotide sequence, identifier tag sequence X, and sequences involved in trimming the amplification product. **D.** Trimming the amplification product to generate tagged single-stranded DNA molecule of defined sequence and length, containing target nucleotide sequence A and identifier tag sequence X. **E.** Incubation of tagged DNA molecule with universal chip. **F.** Hybridization of tagged molecule containing identifier tag X, to complementary detection probe X' on universal chip. **G**. Readout of hybridization by electrochemical detection of Ru(III) complex bound electrostatically to phosphodiesters.

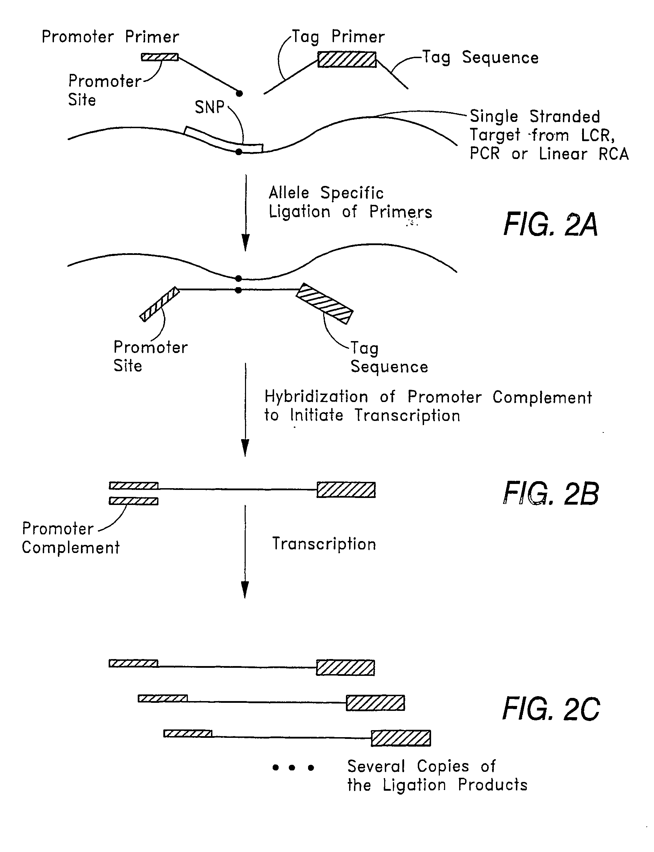

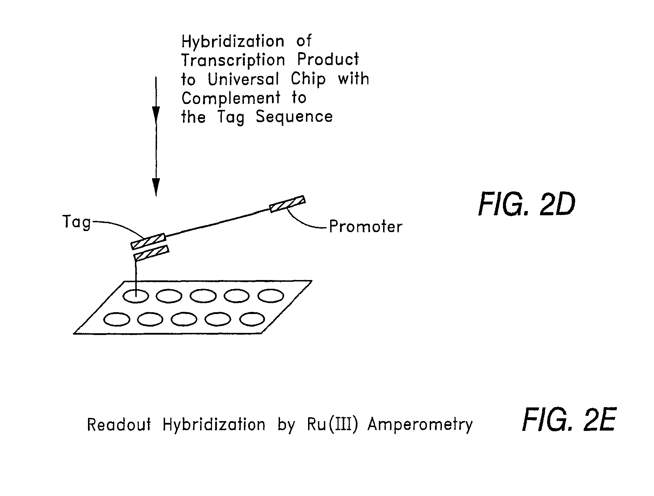

**Figure 2****. A.** Allele-specific ligation of primers on a single-stranded target sequence that can be produced by methods such as PCR, LCR, or linear RC amplification. One primer (promoter primer) has a target-specific region connected to a promoter sequence; the other (tag primer) has a target-specific region connected to an identifier tag sequence. Allele-specific ligation joins the tag primer and promoter primer to form a ligation product having tag sequence and promoter sequence. **B.** A promoter oligonucleotide hybridizes to the promoter sequence in the ligation product to provide a double-stranded site on which to initiate transcription. **C.** Transcription of the ligation product produces several copies of the identifier tag sequence. **D.** Products of transcription are exposed to a universal chip for hybridization of the identifier tag to a complementary detection probe on the chip. **E.** The hybridization of tag sequence would be measured by electrochemical detection of Ru(III) complexes bound electrostatically to phosphodiesters.

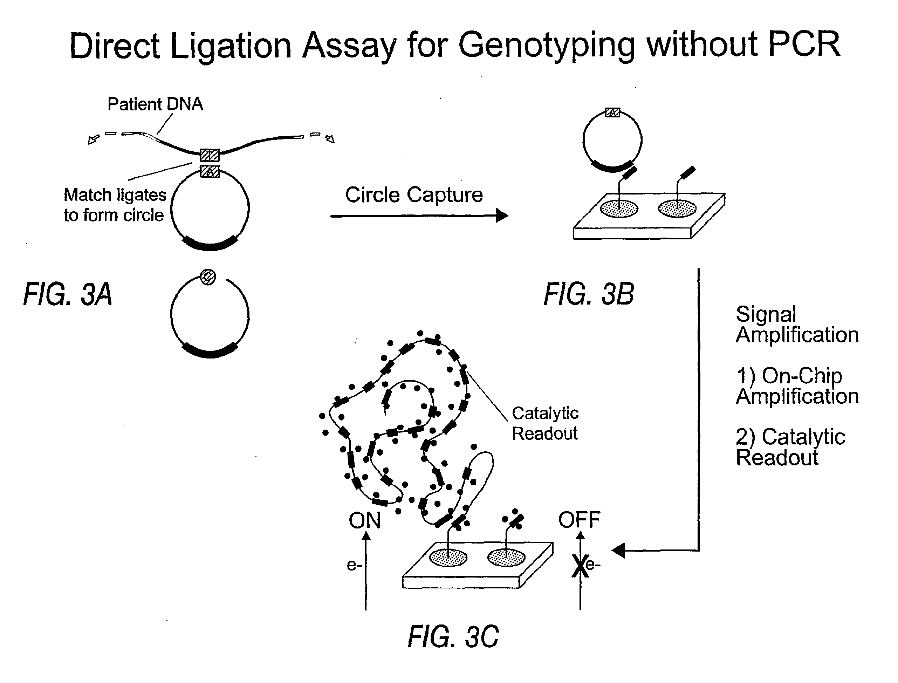

**Figure** 3. **A.** Allele-specific ligation of a linear RC probe to form a circular RC padlock probe having an identifier tag sequence located therein. **B.** The circular RC padlock probe captured on a detector by binding the identifier tag to a detection probe. **C.** Amplification from the free 3' end of the detection probe using the RC probe as a template, thereby forming a single stranded nucleic acid corresponding to multiple catenated copies of sequence complementary to the RC probe. The extended nucleic acid sequence is then detected by amperometry.

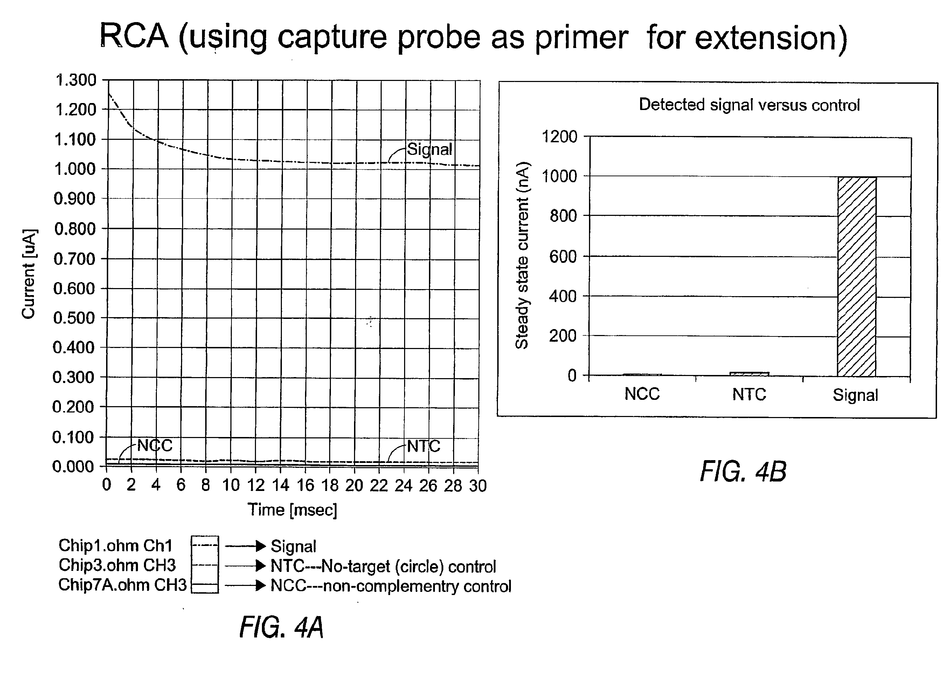

**Figure 4****. A.** Plot of current over time detected by amperometry for signal generated using RC probe complementary to a detection probe (signal), no RC probe control (NTC), and RC probe non-complementary to detection probe (NCC). **B.** Chart comparing the current generated using RC probe complementary to a detection probe (signal), no RC probe control (NTC), and RC probe non-complementary to detection probe (NCC).

### Detailed Description of the Preferred Embodiment

The present disclosure provides methods and compositions for detecting targets in a sample using a universal tag assay. Further provided are universal tag assays and kits for universal tag assays. The present disclosure provides methods and compositions for detecting targets in a sample using a universal detector having detection probes complementary to identifier tags that correspond to known targets, where detection probes are coupled to detection means to measure hybridization of identifier tags that correspond to known targets. In the universal tag assay disclosed herein, tagged molecules having identifier tags are incubated with a universal detector having detection probes coupled to detection means, and hybridization of a particular identifier tag to its complementary detection probe indicates the presence of the corresponding target in the sample being assayed. The universal tag assay disclosed and claimed herein utilizes target-dependent procedures to generate tagged molecules, advantageously increasing accuracy and minimizing spurious signals without the need to employ special conditions or special reagents. The universal tag assay can easily be used to assay a wide variety of samples. The universal tag assay can be performed in a single vessel and easily be automated.

The term "target" as used herein refers to a molecule such as a polynucleotide, polypeptide, small organic molecule, or other molecule of interest in a particular application. The term "target" may also refer to information encoded by a molecule, *e.g.*, polynucleotide sequence, secondary structure determined by the sequence of a polynucleotide, hybridization behavior determined by the sequence of a polynucleotide, amino acid sequence of a polypeptide, secondary or tertiary structure determined by the amino acid sequence of a polypeptide, physical properties determined by the amino acid sequence of a polypeptide, ligand binding sites determined by the sequence of a polypeptide, information about homology or phylogeny found in a nucleotide or amino acid sequence, novel properties of a small molecule determined by molecular structure of the small molecule, and any other information useful in a particular application.

A "target nucleotide sequence" is a nucleotide sequence of interest in a particular application. Generally, the target nucleotide sequence is a portion of the nucleotide sequence of a polynucleotide sometimes referred to as a "target polynucleotide" although when a nucleotide sequence is the target of interest, the term "target nucleotide sequence" is used instead of the term "target polynucleotide." One of skill in the art would understand that the universal tag assay of the present invention detects the presence of a target polynucleotide in a sample by detecting the target nucleotide sequence contained therein. One of skill would understand that a target nucleotide sequence for any particular application is a nucleotide sequence containing sufficient information to identify the target of interest in a particular application. In an embodiment in which multiple targets are being assayed, target nucleotide sequences for each of the multiple targets may be the same or different lengths.

In an illustrative embodiment, a gene has a single nucleotide polymorphism (SNP) with two variant sequences, wherein each variant sequence is associated with a known phenotype. In such an embodiment, a suitable target nucleotide sequence would be a nucleotide sequence having sufficient information capacity to reliably identify which SNP variant sequence or sequences are present in a sample from an individual. For example, a 10-nucleotide target nucleotide sequence may have sufficient information content to reliably identify which variant or variants of the SNP of interest are present in a sample containing copies of the entire genome of the individual. That is, the 10-nucleotide target nucleotide sequence can reliably identify the SNP of interest, against the background of the entire genome. In such a case, the "target" can be variously defined: the target may be the SNP variant sequence of interest, in which case the target is smaller than the target nucleotide sequence; alternately, the target may be considered to be the gene having the SNP(s) of interest, or the individual having a particular SNP genotype for that gene, or the individual having the phenotype associated with the SNP of interest, *u.s.w*. Regardless of how the target is defined, one of skill in the art would understand that a target nucleotide sequence is selected to have sufficient information content to reliably identify the target of interest in a particular embodiment, and may contain additional information beyond the minimum required.

A "tagged molecule" contains an identifier tag for a particular target and may optionally contain a copy or complement of the target. A tagged molecule may contain additional sequence. A tagged molecule is a molecule that interacts with the universal detector as follows: the tagged molecule containing an identifier tag is incubated with the universal detector having detection probes, and the identifier tag in the tagged molecule hybridizes to a complementary detection probe of the universal detector. Tagged molecules are generated by target-dependent processes, such that a tagged molecule containing an identifier tag is generated only when the target corresponding that identifier tag is present in the sample. Preferably, an "identifier tag" is an oligonucleotide having a known nucleotide sequence called the "tag sequence" or "identifier tag sequence." A tagged molecule may be a "tagged target" that contains a copy or complement of the target and an identifier tag for that target. Alternately, a tagged molecule may contain an identifier tag for a particular target and no copy or complement of the corresponding target. A tagged molecule having only the identifier tag and no copy or complement of the target may be generated by cleaving the products of various target-dependent processes to release tagged molecules having only the identifier tag. Alternately, a tagged molecule having only the identifier tag and no copy or complement of the target may be generated by target-dependent processes that only generate copies of the identifier tag. In one embodiment, target nucleotide sequence is amplified, and tagged amplification products having at least one copy of the target and at least one identifier tag are trimmed to generate a smaller tagged molecule containing only the identifier tag. In another embodiment, target-depending binding of a primer or probe generates a tagged product that can be trimmed to release an identifier tag. An identifier tag suitable for use in the universal tag assay is generated only when the corresponding target is present in a sample being interrogated. Thus, a tagged molecule that contains an identifier tag and does not contain a copy or complement of the target is sufficient to indicate the presence of the corresponding target in the sample being assayed.

Identifier tags in tagged molecules suitable for use with a universal detector of the universal tag assay may be DNA or RNA oligonucleotides, and may include modified bases, non-naturally bases, and labels. Generally, tagged molecules are oligonucleotides or polynucleotides (depending on length) that may include modified bases or non-naturally occurring bases, and may additionally include labels, ligands or other materials and modifications suitable to a particular application. Tagged molecules for use with a universal detector can be generated from any suitable template including but not limited to genomic DNA, cDNA, PCR products, LCR products, RC amplification products, synthetic DNA, other forms of DNA, mRNA, rRNA, synthetic RNA, and other forms of RNA. Advantageously, the use of identifier tags and a universal detector having complementary detection probes provides a universal tag assay that is independent of the organism or tissue being analyzed. Multiple target nucleotide sequences can be detected simultaneously, due to the one-to-one correspondence between each identifier tag and the target nucleotide sequence for which it serves as an identifier, and due to the specificity of hybridization of each identifier tag to its detection probe.

### A. Tags and probes

One aspect of the disclosure provides a set of tags and probes for use in accordance with the methods and compositions herein disclosed. Detection probes used with universal detectors of the present invention are directed to complementary tags that serve as identifiers for targets. Likewise, tags that serve as identifiers for targets are directed to complementary detection probes used with universal detectors of the present invention. Hybridization of a tag to its complementary detection probe on a universal detector generates a signal that indicates the presence of the corresponding target known to be identified by that tag. Accordingly, a sample can be interrogated for the presence of targets of interest using tags and probes of the present invention as follows: a) tags are chosen such that each tag serves as an identifier tag for one target; b) a tag capable of hybridizing to a complementary detection probe will be generated only if the sample being interrogated contains the particular target for which that tag serves as an identifier tag; and c) only a tag generated as a result of the presence of the corresponding target in the sample will hybridize to a detection probe and generate a signal on a universal detector.

One of skill in the art would understand that a set of tags and probes is chosen such that each tag to be used as an identifier tag in a particular application has a complementary detection probe on the universal detector being used in that application. One of skill in the art would also understand that the universal tag assay may be practiced using a set of detection probes that includes detection probes complementary to tags that are not being used in a particular application. For example, a universal detector may advantageously be manufactured with a fixed array of 1000 detection probes for use in a wide variety of applications, while a particular application using that universal detector may only use 50-100 identifier tags to interrogate a sample.

It is understood that not only does measuring hybridization of a tag to its complementary detection probe reliably indicate the presence of the corresponding target in a sample, but the absence of hybridization of a tag to its complementary detection probe can also reliably indicate the absence of the corresponding target in a sample. Preferably, at least one internal control is included in a universal tag assay, such that reliably obtaining the expected result from the internal control(s) supports the reliability of results indicating the either the presence or absence of a tag hybridization signal. Multiple internal controls may be used to increase the reliability and robustness of an assay.

Tag/probe sets may include control sequences that may be used for calibration, quality control, and comparison between experiments. Control sequences may include constant sequences or "housekeeping" sequences that are expected to be present in a sample and produce tagged molecules. If desired, the robustness of the assay may be enhanced by choosing more than one distinct tag to serve as an identifier tag for the same target. Advantageously, hybridization of all identifier tags corresponding to the same target to their complementary detection probes would more reliably indicate the presence of the target in the sample being assayed. Likewise, if none of the identifier tags corresponding to the same target hybridize to their complementary tags (especially if other internal controls give positive hybridization signals that indicate suitable reaction conditions), then such a signal more reliably indicates the absence of the target in the sample being assayed. Intermediate results wherein only a few of the identifier tags bind could serve as a signal that reagents or reaction conditions should be examined.

As used herein, the term "tag" generally refers to a molecule capable of binding to a probe, where "tag" may encompass tag molecules attached to a target molecule, tag molecules not attached to target molecules, tags expressed in computer-readable form, and the concept of tags as disclosed herein. The term "tag sequence" as used herein refers to the nucleotide sequence of an oligonucleotide tag, where "tag sequence" or "identifier tag sequence" may describe a string of nucleotides or may describe an information string representing the properties of the string of nucleotides, where such an information string can be manipulated as part of a program for designing or selecting a set of tags having desired properties; preferably, the information string is in computer-readable form. In the present invention, an "identifier tag" is a tag chosen to serve as a distinct identifier for a particular target. As used herein, the term "identifier tag" is used to refer both to the oligonucleotide that binds to a complementary detection probe and to nucleotide sequence of the identifier tag. The term "complement of an identifier tag" can refer to a string of nucleotides that make up the oligonucleotide having a nucleotide sequence complementary to the nucleotide sequence of the identifier tag, and can also refer to the nucleotide sequence (information string) of the complement.

As used herein, the term "detection probe" generally refers to a molecule capable of binding to a tag, where "detection probe" may encompass probe molecules immobilized to a support, probe molecules not immobilized to a support, probes expressed in computer-readable form, and the concept of detection probes as disclosed herein. More specifically, the term "probe sequence" as used herein refers to the nucleotide sequence of an oligonucleotide probe, where "probe sequence" may describe a physical string of nucleotides that make up a sequence, or may describe an information string representing the properties of the string of nucleotides, where such an information string can be manipulated as part of a program for designing or selecting a set of probes having desired properties; preferably, the information string is in computer-readable form. The term "detection probe" is generally used herein to refer to a tag-complementary probe coupled to a detection means for measuring hybridization of a tag to the detection probe. Preferably, a detection probe is immobilized to a support that includes a detection means. Such a support may include but is not limited to a surface, a film, or a particle, where a surface is preferably a "chip" surface suitable for mounting an array of immobilized probes and having at least one component of the detection means, and a particle is preferably a bead having at least one component of the detection means. "Detection probe" can also refer to a computational model of a tag-complementary probe coupled to a detection means for detecting hybridization. The term "detection probe" may particularly be used herein to distinguish the detection probe from other components also referred to by the term "probe" e.g., RC probes and RC padlock probes.

In accordance with one aspect of the present disclosure a set of tag sequences and probe sequences is selected such that a tag having a certain tag sequence will hybridize only to a probe having a sequence that is an exact complement, and no tag will detectably hybridize with any other probe in the set that is not its exact complement. Such a set is referred to herein as a "minimally cross-hybridizing set." It is understand that due to complementarity, a minimally cross-hybridizing set of tag sequences and probe sequences may be selected on the basis of tag sequence or probe sequence. Preferably, all tag sequences in a set are selected to have the same or substantially the same G-C content, such that all probe/tag duplexes have similar melting temperatures. Preferably, tag sequences are selected such that all probe/tag duplexes have similar stacking energy. Advantageously, such a set will provide tag-probe hybridization reactions with the desired level of selectivity. Even more preferably, such selective hybridization reactions can be carried out under conditions of moderate stringency.

The length of tag (and probe) sequences suitable for a given embodiment can be determined by one of skill in the art. Preferably, the length of tag and probe sequences is determined by the size of the tag/probe set used to interrogate a sample. Generally, the size of the tag/probe set used to interrogate a sample will determine the degree of complexity needed, and tag/probe length is an important determinant of complexity. Generally, the estimated number of targets being tested in a sample will determine the size of the tag/probe set needed for that embodiment. A set of tags and probes suitable for use in the universal chip system may include tags and probes of different lengths, as long as all tags and probes satisfy the hybridization criteria for a given embodiment For embodiments involving low density arrays wherein about 100 or fewer targets are to be detected, tags having a length of 10, 11, 12, 13, 14, 15, 16, 17, or 18 nucleotides may be utilized. Preferably, a tag sequence for a low-density array is 15 nucleotides in length. Tags longer than 18 nucleotides may be used for low density arrays if desired. For embodiments involving higher density arrays wherein hundreds or thousands of target sequences are to be detected, tag and probe sequences may need to be greater than 15 nucleotides in length, in order to provide a sufficiently large set of tags and probes that satisfy the hybridization criteria for a given embodiment.

Algorithms for generating minimally cross-hybridizing sets of tags and probes are known in the art. A set of tags and probes having desired properties may be obtained by following some or all of a series of tag selection steps, as follows: a) determining all possible tag sequences of a selected length, and/or all possible tag sequences with selected hybridization properties b) selecting tag sequences so that all tags differ by at least two nucleotides in the tag sequence string, such that no tag can hybridize to a non-complementary probe with fewer than two mismatches; c) if desired, refining the selection based on the relative destabilizing effects of mismatches at different positions; d) selecting tag sequences so that there is no secondary structure within the complementary probes used to detect the tags; e) selecting tags so that probes complementary to the tags do not hybridize to each other; f) when all tags are the same length, selecting tags so that all tags have substantially the same, and preferably exactly the same, overall base composition (*i.e*., the same A+T to G+C ratio), so all tag/probe pairs have the same melting temperature; g) when tags are differing lengths, selecting tags having the A+T to G+C ratio that permits all tag/probe pairs to have the same melting temperature. Additional steps not recited here may also be appropriate to obtain a set of tags and probes having desired properties suitable for a particular embodiment.

Selection steps such as those recited above may be performed in various art-recognized ways. Approaches to designing tag/probe sets for use in a particular application include computational *"in silico"* approaches to model tag and probe behavior, or experimental *"in vitro"* approaches using biomolecules such as polynucleotides to accomplish tag and probe sorting, or combinations of these approaches.

Computational approaches can be used in which computational algorithms serve as models of biological molecules. Such approaches and algorithms are known in the art. For example, computer programs installed on computers can be used to make the relevant calculations and comparisons, to execute a desired set of selection steps, and to generate a suitable set of sequence tags. Methods for applying a series of selection steps to design a tag/probe set can be found in the art, *e.g.,* as disclosed by Morris *et al.* (U.S. Pat. No. 6,458,530 and EP 0799897) where a pool of potential tags is generated and a series of pairwise comparisons is carried out to yield a final set of tags that satisfy certain selection criteria. Open-ended computational approaches such as genetic algorithms to generate (locally) optimized populations may be used.

In a preferred embodiment, a universal chip for use in the universal tag assay includes an array of electrically coupled detection probe sequences lacking G (guanosine) bases, thereby permitting electrochemical detection of hybridization of tagged DNA or RNA molecules by detecting G oxidation in tagged molecules (containing G) bound to detection probes, using methods for detecting oxidation-reduction known in the art. For example, G-oxidation in tagged molecules may be detected using transition metal complexes, preferably ruthenium complexes, as disclosed in U.S. Pat. No. 5,871,918. Advantageously, the use of redox-inactive detection probes (e.g., probes lacking G) permits a high density of probes on a universal detector without a background oxidation signal.

### B. Universal detector

An object of the present disclosure provides a universal detector having detection probes complementary to identifier tags, where detection probes are coupled to a detection means and the interaction of identifier tags with complementary detection probes indicates the presence or absence of targets in the sample being interrogated. Preferably, a universal detector has an array of detection probes. An "array" is a collection of probes in a known arrangement, and an "array of detection probes" as disclosed herein provides a medium for detecting the presence of targets in a sample based on rules for matching tags and probes, where the rules for matching tags and probes are peculiar to each embodiment. Generally, an array of detection probes refers to an array of probes immobilized to a support, where the sequence (the identity) of each detection probe at each location is known. Alternately, an array of detection probes may refer to a set of detection probes that are not immobilized and can be moved on a surface, or may refer to a set of detection probes coupled to one or more particles such as beads. Preferably, the process of detecting identifier tags hybridized to detection probes is automated. Microarrays having a large of number of immobilized detection probes of known identity can be used for massively parallel gene expression and gene discovery studies. A variety of detection means for measuring hybridization of tags to probes are known in the art, including fluorescent, colorimetric, radiometric, electrical, or electrochemical means.

A further object of the present disclosure provides a "universal chip" where the term "universal chip" refers generally to a support having arrays of detection probes selected as described above, wherein the detection probes are coupled to a detection means and further wherein hybridization of tags to probes can be detected. In a preferred embodiment, a detection means utilizes electrochemical detection of hybridization of tags to detection probes immobilized to a "universal chip" in a known array. Because the sequence of each detection probe at each location in such an array is known, the sequence of the complementary identifier tag hybridizing to a detection probe is automatically known and thus, the presence of the target corresponding to that tag is known.

Diverse methods of making oligonucleotide arrays are known, for example as disclosed in U.S. Pat. Nos. 5,412,087, 5,143,854, or 5,384,261 and accordingly no attempt is made to describe or catalogue all known methods. One object of the present invention provides a universal detector having detection probes attached to a support that functions as an electrical contact surface or electrode to detect hybridization of tags to detection probes. Methods for attaching oligonucleotides to an electrical contact surface are well known, for example as disclosed in any of U.S. Pat. Nos. 5,312,527, 5,776,672, 5,972,692, 6,200,761, or 6,221,586.

In the fabrication process, many other alternative materials and processes can be used. The substrate may be glass or other ceramic material; the bottom silicon dioxide can be replaced by silicon nitride, silicon dioxide deposited by other means, or other polymer materials; the conducting layer can be any appropriate material such as platinum, palladium, rhodium, a carbon composition, an oxide, or a semiconductor. For amperometric measurement either a three-electrode system consisting working electrode, counter electrode and reference electrode or a two-electrode system consisting working and a counter/reference electrode is necessary to facilitate the measurement. The working electrodes should provide a consistent surface, reproducible response from the redox species of interest, and a low background current over the potential range required for the measurement. The working electrodes may be any suitable conductive materials, preferably noble metals such as gold and platinum, or conductive carbon materials in various forms including graphite, glassy carbon and carbon paste. For a three electrode system the reference electrode is usually silver or silver/silver chloride, and the counter electrode may be prepared by any suitable materials such as noble metals, other metals such as copper and zinc, metal oxides or carbon compositions. Alternatively, the conducting layer can be prepared by screen printing of the electrode materials onto the substrate. Screen printing typically involves preparation of an organic slurry or inorganic slurry of an electrode material, such as a fine powder of carbon or gold, onto the substrate through a silk screen. The electrode material slurry may be fixed on the surface by heating or by air drying. The electrode may be any suitable conductive material such as gold, carbon, platinum, palladium, indium-tin-oxide. It is often advantageous to coat the electrode surface with a material such as avidin, streptavidin, neutravidin, or other polymers, to increase the immobilization of detection probes. Methods for the attachment include passive adsorption and covalent attachment.

If gold is chosen for the conducting layer, the layer can be evaporated, sputtered, or electroplated. A low temperature oxide layer can be replaced by spin-on dielectric materials or other polymer materials such as polyimide, or parylene. Reagent and electrical connections can be on the same side of a chip or on adjacent sides, though the opposite side configuration is preferred. Materials, temperatures, times, and dimensions may be altered to produce detectors, preferably chips, having substantially the same properties and functionality, as will be appreciated by those of skill in the art. Materials, temperatures, times, and dimensions may be altered by one of skill in the art to produce chips having the properties desired for any particular embodiment.

In a preferred embodiment of the disclosure the detection probes are immobilized on a support having an array of electrodes sandwiched between two layers of silicon dioxide insulator attached to the silicon substrate., where a supporting layer is opposite the silicon substrate and the chip is oriented such that the silicon substrate is on the top and the supporting layer is on the bottom, as disclosed in U.S. Pat. Application No. 10/121,240. Preferably, gold electrodes are used. Alternately, carbon electrodes such as graphite, glassy carbon, and carbon paste can be used. In this preferred embodiment, access to the surfaces of the working electrodes, where the detection probes are immobilized, is through windows through the silicon substrate and top layer of insulator on the top surface of the chip. Windows on the underside (etched through the supporting layer and the bottom layer of insulator) allow access to a counter (or detector) electrode and a reference electrode. For gold electrodes, the two types of electrodes in the chip are selectively interconnected by deposited gold wiring within the insulating layer or by other methods known in the art. Access to the working electrode, reference electrode, and counter electrode allows a complete circuit to be formed which will enable standard techniques in the art, such as amperometric measurements, to be performed using the chip. An electrode potential applied to the working electrode, where the electrochemically active materials are present through association with the detection probes and tag sequences, will produce current proportional to the amount of tag sequence attached to the detection probes.

### B.1. Detection means/Detection components: Measuring hybridization of identifier tags to complementary detection probes

Another aspect of the disclosure provides detection means or components for measuring hybridization of tags to detection probes. In one embodiment, DNA hybridization is detected by an electrochemical method, which generally includes observing the redox behavior of a single-stranded DNA detection probe as compared to a double-stranded DNA. For example, a voltammetric sequence-selective sensor can be used for detecting a target nucleic acid, where a double-stranded nucleic acid is contacted to a redox-active complex for example as disclosed in U.S. Pat. No. 5,312,527. The complex binds non-specifically to the double-stranded DNA, and because the complex itself is the redox-active compound that provides a voltammetric signal, the complex does not function in a catalytic manner without the addition of an enzyme. Alternately, an electrochemical assay for nucleic acids can be used, in which a competitive binding event between a ligand and an antiligand is detected electrochemically, as disclosed in U.S. Pat. No. 4,840,893.

In another embodiment, RNA hybridization is detected by an electrochemical method, which generally includes observing the redox behavior of a single-stranded DNA detection probe as compared to a DNA/RNA duplex formed by hybridization of an RNA tag to a DNA detection probe.

Hybridization of tags and probes may be detected using a transition metal complex capable of oxidizing at least one oxidizable base in an oxidation-reduction reaction under conditions that cause an oxidation-reduction reaction between the transition metal complex and the oxidizable base, where the probe or the tagged molecule or both contain at least one oxidizable base. The oxidation-reduction reaction indicating hybridization is detected by measuring electron transfer from each oxidized base, as disclosed in U.S. Pat. No. 5,871,981.

In a preferred embodiment, hybridization of identifier tags to DNA detection probes immobilized on gold or other electrodes may be carried out using methods disclosed by Steele et al. (1998, Anal. Chem 70:4670-4677). Preferably, multivalent ions with 2, 3, or 4 positive charges are used, which are capable of electrochemical detection by direct reaction without affecting the nucleic acid. In the preferred embodiment these ions bind electrostatically to nucleic acid phosphate irrespective of whether it is in the double-helical or single-stranded form. The presence or absence of hybridized identifier tag DNA is determined for each detection probe, based on electron transfer measurements taken at each detection probe site. The sample being interrogated may be contacted with the oligonucleotide detection probe in any suitable manner known to those skilled in the art. By way of example, a DNA sample being interrogated for the presence of target nucleotide sequences may be in solution and the oligonucleotide detection probes immobilized on a solid support, whereby the DNA sample may be contacted with the oligonucleotide detection probe by immersing the solid support having the oligonucleotide detection probes immobilized thereon in the solution containing the DNA sample. Suitable transition metal complexes that bind nucleic acid electrostatically and whose reduction or oxidation is electrochemically detectable in an appropriate voltage regime include Ru(NH₃)₆³⁺, Ru(NH₃)₅pyridine³⁺ and other transition metal complexes that can be determined by one of skill in the art.

In accordance with another aspect of the present disclosure, oligonucleotide detection probe sequences may be designed to be redox inactive, or to have very low redox activity, for example as disclosed in U.S. Pat. No. 5,871,918. In one embodiment, oligonucleotide probe sequences are designed so as to not contain G (guanosine) bases, permitting electrochemical detection of hybridization of tagged DNA molecules by detecting G oxidation in tagged molecules with identifier tags hybridized to their probe complements, as disclosed in U.S. Pat. No. 5,871,918. Advantageously, the use of redox-inactive probes permits a high density of probes on a universal detector without a background oxidation signal.

The occurrence of the oxidation-reduction reaction may be detected according to any suitable means known to those skilled in the art. For example, the oxidation-reduction reaction may be detected using a detection electrode to observe a change in the electronic signal which is indicative of the occurrence of the oxidation-reduction reaction. Suitable reference electrodes will also be known in the art and include, for example, silver, silver/silver chloride electrodes. The electronic signal associated with the oxidation-reduction reaction permits the determination of the presence or absence of hybridized tags by measuring the Faradaic current or total charge associated with the occurrence of the oxidation-reduction reaction. The current depends on the presence of the positively charged redox ion closely associated with the electrode, which in turn depends on the amount of nucleic acid phosphate hybridized to the electrode. The electronic signal may be characteristic of any electrochemical method, including cyclic voltammetry, normal pulse voltammetry, differential pulse voltammetry, chronoamperometry, and square-wave voltammetry. The amount of hybridized DNA is determined by subtracting the current or total charge characteristic of the probes and other molecules bound to the electrode in the starting state from the current or total charge measured after the hybridization reaction.

It will be appreciated that in addition to the amperometric methods described above, methods that are well known in the art can be used to detect the hybridization of identifier tags to detection probes. Such methods include, but are not limited to, hybridization methods using labeled nucleic acid probes, antibody based detection and enzymatic detection. For example, in some embodiments, a signal tag can be included in a nucleic acid having an identifier tag. As used herein, "signal tag" means a nucleic acid sequence that is complementary to a detectable signal probe. If the identifier tag binds to the detection probe, the signal tag sequence will also be present on the detector. A signal probe that is complementary to a signal tag can be generated by internal or end labeling with ³²P or ³³P. Alternatively, light emitting molecules or other non-radioactive labels can be used to generate labeled signal probe. Hybridization of the labeled signal probe to the signal tag sequence can be detected using autoradiography. In another embodiment, the signal probe is a nucleic acid comprising chemical modifications which can be detected through the use of an antibody. For example, the signal probe can be labeled with fluoroscein and the fluoroscein can be detected using an antifluoroscein antibody. In still other embodiments, an enzyme such as horseradish peroxidase or alkaline phosphatase can be directly linked to the signal probe.

It will be appreciated that numerous variations of the above methods for detecting the hybridization of nucleic acids are well known in the art. Accordingly, a skilled artisan would recognize such detection methods, although not described above, are well within the scope of the present invention.

### C. Preparation of tagged molecules

Another aspect of the present disclosure provides tagged molecules generated by target-dependent processes, where tagged molecules containing identifier tags are generated only in the presence of target. Advantageously, the tag/probe sets and universal detector of the present invention provide convenient, resource-efficient materials and methods for designing and detecting tagged molecules, while target-dependent generation of tagged molecules substantially decreases or entirely eliminates the possibility of false positive signals. In a preferred embodiment, tagged molecules containing identifier tags are generated by manipulation of a template containing target nucleotide sequence. Amplification of template containing target nucleotide sequence can generate tagged molecules containing the identifier tag(s) corresponding to the target nucleotide sequence. Target-dependent probe or primer binding can also generate tagged molecules containing the identifier tag(s) corresponding to a target.

As used herein, "template" refers to all or part of a polynucleotide containing at least one target nucleotide sequence. As described above, "target nucleotide sequence" refers to the nucleotide sequence of interest in a particular application. An "exogenous nucleotide sequence" as used herein, refers to a sequence introduced during preparation of tagged molecules. The presence an identifier tag or target nucleotide sequence (or copy, complement, or portion thereof) is specifically referred to in the present disclosure, such that "exogenous nucleotide sequence" or "additional exogenous nucleotide sequence" generally refers to nucleotide sequence not found in target nucleotide sequence and identifier tag sequence. When exogenous nucleotide sequence includes sequence(s) normally found in the sample or organism from which the sample is obtained, the exogenous nucleotide sequence will be found be in an arrangement not found in the original template from which the target nucleotide sequence was copied. Preferably, exogenous nucleotide sequence is introduced by primers or probes used in target-dependent processes involved in generating tagged molecules suitable for use in the universal tag assay.

As used herein, an "auxiliary oligonucleotide" is an oligonucleotide, preferably DNA or RNA, that can be used to create a region of double-stranded DNA or RNA, or DNA/RNA heteroduplex, by incubating a single-stranded polynucleotide with an auxiliary oligonucleotide complementary to a portion of sequence on the single-stranded polynucleotide. Auxiliary nucleotides can be used to create localized regions of double-stranded DNA, RNA, or DNA/RNA to generate a restriction digestion site that permits cleavage of the single-stranded polynucleotide, or a polymerase promoter that permits polymerase binding and copying of the single-stranded polynucleotide. Auxiliary oligonucleotides can function as polymerization primers, including for rolling circle (RC) amplification. In a preferred embodiment, auxiliary oligonucleotides are complementary to one or more portions of single-stranded amplification products containing target nucleotide sequence and identifier tag sequence, and form regions of DNA duplex that create a restriction digestion site that enables trimming of the single-stranded amplification product to generate a smaller tagged molecule. Auxiliary oligonucleotides and primers may contain chemical modifications to enable trimmed single-stranded product(s) to be separated from primers and auxiliary oligonucleotides. In a preferred embodiment, the chemical modification is an addressable ligand permitting recovery of a molecule containing the ligand. In a more preferred embodiment, the addressable ligand is a biotin residue.

In accordance with another aspect of the present disclosure, the template may be any polynucleotide suitable for amplification, where the template contains at least one target nucleotide sequence to be amplified. Suitable templates include DNA and RNA molecules, and may include polynucleotides having modified bases. Preferably, templates are genomic DNA molecules, cDNA molecules, PCR products, LCR products, synthetic (synthesized) DNA molecules, other forms of DNA, mRNA molecules, rRNA molecules, synthetic (synthesized) RNA molecules, or other forms of RNA. Methods disclosed herein, in particular rolling circle (RC) amplification, can be used to amplify RNA templates directly without reverse-transcribing RNA template into DNA. If necessary, single-stranded template can be obtained by denaturing double-stranded DNA to generate single-stranded template, preferably target strand containing at least one target nucleotide sequence and complementary-target strand containing at least one complement of target nucleotide sequence. The double-stranded DNA may be genomic DNA.

### C.1. Amplification of template

In accordance with one aspect of the disclosure generating tagged molecules suitable for use in the universal tag assay involves amplification of templates using well-known methods to generate amplification products including at least one target nucleotide sequence and at least one identifier tag. Optionally, amplification products contain additional exogenous nucleotide sequences involved in post-amplification manipulation of the amplification product without a significant effect on the amplification step itself. Suitable templates include DNA and RNA molecules such as genomic DNA, cDNA, and mRNA. Linear or exponential (nonlinear) modes of amplification may be used with any suitable amplification method, where choice of mode is made by one of skill in the art depending on the circumstances of a particular embodiment. Methods of amplification include, but are not limited to, use of polymerase chain reaction (PCR) and rolling circle (RC) amplification to amplify polynucleotide templates.

One aspect of the disclosure provides an amplification-dependent method for detecting a target nucleotide sequence in a sample, where the method includes but is not limited to the following steps: a) obtaining template having the target nucleotide sequence; b) amplifying the template to generate at least one tagged molecule including at least one copy of the target nucleotide sequence and at least one identifier tag for the target nucleotide sequence; c) incubating at least one tagged molecule with a universal detector having detection probes coupled to a detection means; c) detecting hybridization of identifier tags to complementary detection probes on a universal detector. Amplification products of step b) have multiple repeating copies of the target nucleotide sequence and at least one identifier tag for the target nucleotide sequence, and can function as tagged molecules suitable for the universal tag assay. Alternately, amplification products of step b) have additional exogenous nucleotide sequences including sequences involved in trimming amplification products, and amplification products are trimmed generate at least one smaller tagged molecule suitable for use in the universal tag assay. In one embodiment, each trimmed tagged molecule contains a copy of the target and an identifier tag for the target. In another embodiment, each trimmed tagged molecule contains an identifier tag without a copy of the target corresponding to that identifier tag. Trimmed tag molecules may contain additional sequence. In accordance with this method, detecting hybridization of an identifier tag to a complementary detection probe on the universal detector indicates the presence of the target nucleotide sequence in the sample. Another aspect provides a method for detecting multiple target nucleotide sequences in a sample, wherein each target nucleotide sequence has a distinct identifier tag.

### Amplification using polymerase chain reaction (PCR)

Template amplification by polymerase chain reaction (PCR) uses multiple rounds of primer extension reactions in which complementary strands of a defined region of a DNA molecule are simultaneously synthesized by a thermostable DNA polymerase. During repeated rounds of primer extension reactions, the number of newly synthesized DNA strands increases exponentially such that after 20 to 30 reaction cycles, the initial template can be replicated several thousand-fold or million-fold. Methods for carrying out different types and modes of PCR are thoroughly described in the literature, for example in *"*PCR Primer: A Laboratory Manual" Dieffenbach and Dveksler, Eds. Cold Spring Harbor Laboratory Press, 1995, and by *Mullis et al.* in patents *(e.g.,* U.S. Patent Nos. 4,683,195, 4,683,202 and 4,800,159) and scientific publications (*e.g.* Mullis et al. 1987, Methods in Enzymology, 155:335-350), and in U.S. Patent Application No. 10/138,067.

Briefly, PCR proceeds in a series of steps as described below. In the initial step of the procedure, double-stranded template is isolated and heat, preferably between about 90°C to about 95°C, is used to separate the double-stranded DNA into single strands (denaturation step). The initial denaturation step is omitted for single-stranded template. Cooling to about 55°C allows primers to adhere to the target region of the template, where the primers are designed to bind to regions that flank the target nucleic acid sequence (annealing step). Thermostable DNA polymerase (*e.g., Taq* polymerase) and free nucleotides are added to create new DNA fragments complementary to the target region of the template via primer extension (extension step), to complete one cycle of PCR. This process of denaturation, annealing and extension is repeated numerous times, preferably in a thermocycler. At the end of each cycle, each newly synthesized DNA molecule acts as a template for the next cycle, resulting in the accumulation of many hundreds or thousands, or even millions, of double-stranded amplification products from each template molecule.

In multiplex PCR, the assay is modified to include multiple primer pairs specific for distinct target nucleotide sequences of the same template, to allow simultaneous amplification of multiple distinct target nucleotide sequences and generation of multiple distinct single-stranded DNA molecules having the desired nucleotide sequence and length. For example, multiplex PCR can be carried out using the genomic DNA of an organism or an individual as the template, where multiplex PCR will produce multiple distinct single-stranded DNA molecules.

PCR generates double-stranded amplification products suitable for post-amplification processing. PCR amplification products may contain features such as additional nucleotide sequences not found in the target nucleotide sequence. Primers used to amplify template may be designed to introduce features into amplification products by introducing exogenous nucleotide sequence(s) not found in the target nucleotide sequence. Such features include, but are not limited to, identifier tags, restriction digestion sites, modified nucleotides, promoter sequences, inverted repeats, chemical modifications, addressable ligands, and other non-template 5' extensions that allow post amplification manipulation of amplification products without a significant effect on the amplification itself. Preferably, the exogenous sequences are 5' ("upstream") of the primer sequence involved in binding to the target nucleotide sequence. In one preferred embodiment, primers introduce identifier tags. In another embodiment, of the disclosure primers introduce sites involved in restriction enzyme recognition, binding and cleavage ("trimming") of amplification products.

### Amplification using ligation reactions

In accordance with another aspect of the present disclosure, ligation primers are used in ligation reactions to produce ligation products that include sequence complementary to at least a portion of target nucleotide sequence. In various embodiments, ligation products are suitable for use in subsequent processing or amplification steps. Advantageously, ligation reactions provide a means for target-dependent discrimination. Ligation reactions include but are not limited to the following steps: a) obtaining single-stranded template having at least one target nucleotide sequence; b) contacting the template with a plurality of oligonucleotide ligation primers, where at least one pair of ligation primers is designed to hybridize to at least one target nucleotide sequence on the template, such that the 5' end of one of the pair of ligation primers hybridizes adjacent to the 3' end of the other of the pair of ligation primers; c) incubating template and ligation primers under conditions that promote adjacent hybridization of at least one pair of ligation primers to the target nucleotide sequence on the template and ligation of any adjacent hybridized pair of ligation primers to form at least one ligation product that includes sequence complementary to the target nucleotide sequence; d) dissociating the ligation product from the template; e) repeating the hybridization and ligation steps as desired; and f) recovering the ligation products for use in subsequent processing or amplification steps. Preferably, ligation primers suitable for use in the ligase chain reaction (LCR) are used to produce ligation products. Alternately, ligation primers suitable for use in non-enzymatic ligation reactions may be used to produce ligation products using non-enzymatic means, for example as described by Xu and Kool (1999, Nuc Acids Res 27:875-881). In one embodiment, LCR is repeated using temperature cycling for exponential amplification of the target nucleotide sequence.

In the present disclosure, the term "LCR products" is intended to encompass to ligation products generated by non-enzymatic ligation as well as by enzymatic ligation, specifically by the ligase chain reaction (LCR).

In another embodiment, PCR products containing at least one copy of a target nucleotide sequence are used as template for ligation reactions as described herein. Optionally, PCR products contain at least one exogenous nucleotide sequence introduced by at least one primer.

### Rolling circle methods

In accordance with one aspect of the present disclosure rolling circle (RC) methods can be used for amplification, transcription, target discrimination, and other target-dependent steps. Figure 1 illustrates detecting a SNP in a sample, including the use of RC methods to generate tagged molecules in accordance with the present invention. Protocols for carrying out RC methods are well known in the art, particularly as disclosed by Kool et al. (U.S. Patent Nos. 5,714,320, 6,368,802 and 6,096,880), Landegren et al. (U.S. Patent No. 5,871,921), Zhang et al. (U.S. Patent Nos. 5,876,924 and 5,942,391) and Lizardi *et al.* (Lizardi et al., 1998, Nature Genet 19: 225-232, and U.S. Patent Nos 5,854,033, 6,124,120, 6,143,495, 6,183,960, 6,210,884, 6,280,949, 6,287,824, and 6,344,329). The use of RC amplification to amplify target nucleotide sequences from templates to generate DNA molecules of defined sequence and length is disclosed in U.S. Patent Application No. 10/138,067 and U.S. Provisional Patent Application No. 60/404,195. Advantageously, RC amplification can be carried out as an isothermal amplification method having high specificity and sensitivity for target nucleotide sequences and a low level of nonspecific background signal. Further advantageously, a ligation step in RC amplification can be manipulated to discriminate among variant sequences, for example to carry out allelic discrimination or identify single nucleotide polymorphisms (SNPs), splice variants, mutants, or alleles of a nucleotide sequence.

RC methods require a circular RC probe. Briefly, the first step in practicing RC methods is to generate a linear RC probe, which is a DNA or RNA molecule that can be circularized and ligated to create a functional circular RC probe. Materials and methods for constructions of linear RC probes, or "precircle" molecules, are disclosed by Kool et al. (U.S. Patent Nos. 5,714,320, 6,368,802 and 6,096,880). In order to directly amplify DNA or RNA template containing target nucleotide sequence, the RC probe in linear form is preferably hybridized to denatured template containing target nucleotide sequence, where the 3' and 5' ends of the RC probe have sequences complementary to portions of the target nucleotide sequence. A spacer or "backbone" region is between the 3' and 5' termini, which may include sequences involved in other steps such as binding of polymerization primers, generating identifier tags, detecting/capturing RC probes, or trimming of amplification products. In one embodiment, the 3' and 5' termini hybridize adjacently to target, and the two ends are ligated to form a circularized RC probe suitable for RC amplification. In another embodiment, there is a gap between the two complementary regions when 3' and 5' termini of the linear molecule is hybridized to the target, the gap is filled by primer extension or an auxiliary nucleotide, and the ends are ligated to form the circularized RC probe suitable for amplification. Ligation to form the RC probe may be carried out using enzymatic means, *e.g*., using ligase, preferably T4 ligase, or may be carried out using non-enzymatic protocols, for example as disclosed by Xu and Kool (1999, Nuc Acids Res 27:875-881) and Kool (U.S. Patent Nos. 5,714,320, 6,368,802 and 6,096,880).

Target-dependent hybridization and ligation of an RC probe produces a probe in the "padlock" probe configuration wherein the probe is topologically connected to the target through catenation, *e.g*., as described by Landegren et al. in U.S. Pat. No. 5,871,921.

One aspect of the present disclosure provides RC probes that contain sequence complementary to the target nucleotide sequence, sequence complementary to the sequence of an identifier tag for that target nucleotide sequence, and optionally, additional sequence which may include sequences involved in trimming amplification products, sequences involved in primer binding, sequences involved in detection or capture of probes or products, sequences involved in forming polymerase promoters, and other sequences that provide desired structural or informational features.

A defined set of tags and probes is used in each embodiment, and one identifier tag is associated with each RC probe directed to a distinct target nucleotide sequence, thereby providing a distinct identifier tag for each target nucleotide sequence. The identifier tag sequence is incorporated during synthesis of the RC probe using methods known in the art. For a given embodiment, a defined tag/probe set is selected to have properties required for that embodiment, such as tag/probe length, melting temperature, or complexity. Generally there is no nexus between the tag chosen as the identifier for a target nucleotide sequence and the target identified by that tag. Generally, any tag from a selected tag set can be incorporated into any DNA molecule used to create an RC probe, as long as an accounting is kept of which tag was chosen to serve as the identifier tag for which target nucleotide sequence. One of skill in the art can determine whether, for a given embodiment, any tag from the set can be used with any target nucleotide sequence in any RC probe, or whether constraints exist that would require using certain tags in certain RC probes. One of skill in the art would understand that the universal detector used with any given embodiment will include detection probes complementary to the identifier tag set used in that embodiment, where the detector may additionally include detection probes complementary to tags not used in that embodiment.

Amplification of RC probes according to the methods disclosed herein is catalyzed by a strand-displacing DNA polymerase that generates a single-stranded amplification product containing target nucleotide sequence and identifier tag sequence. A polymerization primer is necessary to initiate RC amplification of DNA products from RC probes, as disclosed by Kool (U.S. Patent Nos. 5,714,320, 6,368,802 and 6,096,880). Amplification products containing multiple copies of the RC probe may be used as tagged molecules suitable for use in the universal tag assay, or may be trimmed to generate smaller tagged molecules suitable for use in the universal tag assay. Optionally, single-stranded amplification product is further amplified to produce double-stranded, preferably hyperbranched, amplification products, for example as disclosed in Zhang et al. (U.S. Patent Nos. 5,876,924 and 5,942,391) and Lizardi *et al*. (Lizardi et al., 1998, Nature Genet 19: 225-232, and U.S. Patent Nos. 5,854,033, 6,124,120, 6,143,495, 6,183,960, 6,210,884, 6,280,949, 6,287,824, and 6,344,329). Double-stranded amplification products containing RNA polymerase promoter regions can be used to drive transcription of DNA amplification product to produce tagged RNA molecules. Preferably, the polymerase is T7 RNA polymerase, *e.g*., using templates disclosed by Milligan et al. (1987, Nuc Acid Res 15:8783-8798). Alternately, T3 or SP6 RNA polymerase or any suitable RNA polymerase may be used with a correct promoter sequence.

In accordance with the methods described herein, an RC probe may contain a sequence involved in forming a site involved in enzymatic digestion of amplification products, such that amplification products contain a recognizable site that permits digestion of the amplification product to produce tagged molecules. Auxiliary oligonucleotides may be required to form regions of duplex that serve as sites for binding and cleaving the amplification product, *e.g*., by restriction endonucleases. The RC probe may optionally include additional sequences involved in further trimming of the smaller tagged molecules produced by enzymatic digestion of amplification products. Sequences involved in further trimming of the tagged molecules produced by enzymatic digestion of the single-stranded amplification product may include self-complementary sequences that form hairpin structures having at least one restriction enzyme recognition site for a restriction enzyme involved in the trimming step. Preferably, restriction enzymes involved in the trimming step may be a Type II or Type IIS restriction enzyme, including *Eco*RI or *Fok*I. Sequences involved in trimming may include sequences that form at least one restriction enzyme recognition site for a restriction enzyme involved in the trimming step upon addition of at least one auxiliary oligonucleotide.

### Amplification of RNA templates to generate DNA molecules

In accordance with another aspect of the present disclosure RC amplification as disclosed and claimed herein may be used to amplify RNA templates to generate single-stranded tagged DNA or RNA amplification products including a copy of complement of target nucleotide sequence and a distinct identifier tag. RNA templates containing target nucleotide sequence may be reverse-transcribed to generate cDNA which may be amplified as described herein. Alternately, RNA template may be amplified directly utilizing an RC probe and RNA-dependent RNA polymerase as disclosed, *e.g*., by Kool (U.S. Patent Nos. 5,714,320, 6,368,802 and 6,096,880).

### Use of ligation products as primers for rolling circle amplification

In a preferred embodiment, of the disclosure each ligation primer containing sequence complementary to a portion of the target nucleotide sequence also contains exogenous nucleotide sequence complementary to a portion of the backbone of an RC padlock probe that contains a copy of the target nucleotide sequence and a complement of the identifier tag sequence for that target nucleotide sequence. The ligation product formed by these primers includes sequence complementary to the target nucleotide sequence flanked by 5' and 3' exogenous nucleotide sequence complementary to a portion of the backbone of the RC padlock probe. The ligation product is then incubated with at least one linear RC padlock probe, under conditions that promote hybridization of the linear RC padlock probe to the ligation product, such that the 5' end of the linear RC padlock probe is adjacent to the 3' end of the linear RC padlock probe and the 5' and 3' ends are ligated to form a circularized RC padlock probe. DNA polymerase is added to the complex formed by the circularized RC padlock probe and the ligation product, under conditions that permit RC amplification of the RC padlock probe using the ligation product as a polymerization primer.

In this embodiment, the amplification product is a single-stranded DNA molecule containing multiple repeating copies of the RC probe, including but not limited to copies of the complement of the target nucleotide sequence, copies of the identifier tag sequence, and copies of any additional sequence found in the RC probe. This amplification product is a tagged molecule suitable for use in the universal tag assay. The amplification product may include modified nucleotides, addressable ligands, sites for enzymatic digestion, or other modifications. In another embodiment, the amplification product additionally contains exogenous nucleotide sequence involved in post-amplification trimming of the amplification product to yield smaller tagged molecules suitable for use in the universal tag assay. In accordance with this aspect of the invention, no additional polymerization primer is needed because the ligation product is completely complementary to the RC padlock probe and thus serves as a polymerization primer for RC amplification when DNA polymerase is added to the reaction mixture.

In another preferred embodiment, a circular RC probe having a copy of target nucleotide sequence and a complement of the identifier tag for that target sequence is incubated with the ligation product containing sequence complementary to the target nucleotide sequence flanked by 5' and 3' exogenous nucleotide sequence complementary to a portion of the backbone of an RC padlock probe. The ligation product hybridizes to the complementary region of the circular RC probe and serves as an polymerization primer for RC amplification when DNA polymerase is added to the reaction mixture. Amplification of the RC probe generates tagged molecules suitable for use in the universal tag assay as described above.

### Use of amplification products to generate tagged molecules

In accordance with another aspect of the present disclosure, amplification products are used in further amplification steps to generate tagged molecules suitable for use in the universal tag assay. Target nucleotide sequence is amplified using PCR or LCR to generate a first amplification product suitable for use as a template. If necessary, double-stranded amplification product is denatured to generate single-stranded template. A single-stranded first amplification product containing a complement of target nucleotide sequence is incubated with a linear RC probe containing a copy of the target nucleotide sequence and a complement of the identifier tag for that target nucleotide sequence. The linear RC probe is catenated to the first amplification product by target-dependent binding, *i.e*., the linear RC probe hybridizes to the region of first amplification product that contains a complement of the target nucleotide sequence, and the 3' and 5' ends of the linear RC probe are ligated to form a circularized RC probe. When the first amplification product is not completely complementary to the RC probe, an additional polymerization primer may be added to drive RC amplification when DNA polymerase is added to the reaction mixture. RC amplification produces a second amplification product containing multiple repeating copies of the RC probe. These second amplification products are tagged molecules suitable for use in the universal tag assay. Optionally, second amplification products are trimmed to generate smaller tagged molecules suitable for use in the universal tag assay. Tagged molecules generated by this method are incubated with a universal detector and hybridization of identifier tags to complementary detection probes is measured. One of skill in the art would understand that this method can also be practiced using a linear amplification product containing a copy of the target nucleotide sequence and an RC probe containing a complement of target nucleotide sequence. Advantageously, this method can be carried out at elevated temperatures to overcome topological constraints associated with RC amplification using padlock probes. Kuhn et al., 2002, Nuc Acids Res 30:574-580.

In one embodiment of the disclosure PCR generates double-stranded linear DNA molecules containing a copy of the target nucleotide sequence. One terminus of the PCR product contains an addressable ligand such as biotin, introduced by primers used for PCR. The linear amplification product is denatured to generate single-stranded PCR products, wherein at least one strand contains an addressable ligand at one terminus. In a preferred embodiment, a biotinylated single-stranded PCR product having a copy of the target nucleotide sequence is incubated with streptavidin-coated beads, under conditions such that the biotinylated PCR product is attached to a bead, forming a bead-target sequence complex. The bead-target sequence complex is incubated with linear RC padlock probes that contain sequence complementary to target nucleotide sequence at their 3' and 5' ends, a complement of the identifier tag for that target nucleotide sequence, and additional RC probe sequence as needed or desired. The RC linear probes hybridize to the bead-target sequence complex in a target-dependent manner as described above, such that the 3' and 5' ends are adjacent and can be ligated as described herein, forming a circularized RC padlock probe. RC amplification of the RC padlock probe generates amplification molecules having repeating copies of target nucleotide sequence and identifier tag. Optionally, an additional primer may be added as a polymerization primer for RC amplification of the padlock probe. These RC amplification products are tagged molecules suitable for use in the universal tag assay, or may be trimmed to generate smaller tagged molecules suitable for use in the universal tag assay. Advantageously, RC amplification of the padlock probe may be carried out at 65° C, preferably using *Bst* DNA polymerase (New England Biolands, Beverly MA). Further advantageously, the RC padlock probe can eventually dissociate from ('fall off) the bead-target sequence complex and continue to be amplified. Alternately, RC transcription of the catenated RC padlock probe using RNA polymerase produces tagged RNA molecules suitable for use in the universal tag assay.

### C.2 Trimming DNA amplification products to generate tagged molecules

One aspect of the disclosure provides that exogenous nucleotide sequence introduced during an amplification step may include sequences involved in trimming the amplification product to produce smaller tagged molecules suitable for use in the universal tag assay. Trimming of amplification products to produce smaller tagged molecules is not required to practice the present invention, and one of skill in the art can determine when a trimming step may be desirable. Methods and compositions for trimming amplification products are disclosed in U.S. Patent Application No. 10/138,067 and U.S. Provisional Patent Application No. 60/404,195. In one embodiment, the exogenous nucleotide sequence may contain self-complementary sequences that form hairpin structures.. These self-complementary sequences that form hairpin structures may contain at least one restriction enzyme recognition site for a restriction enzyme involved in the trimming step, and suitable restriction enzymes include Type II restriction enzymes such as *Eco*RI, or Type IIS restriction enzymes such as *Fok*I. In another embodiment, the exogenous nucleotide sequence may include sequences involved in trimming the amplification product by restriction enzymes, where the exogenous sequence encodes one strand of the restriction enzyme recognition site, and the double-stranded restriction enzyme recognition site is formed upon addition of at least one auxiliary oligonucleotide. Suitable restriction enzymes include Type II restriction enzymes such as *Eco*RI, or Type IIS restriction enzymes such as *Fok*I.

Amplification products may be trimmed to form at least two types of tagged molecules. In one preferred embodiment, a tagged molecule generated by trimming is a tagged target molecule that includes a copy or complement of a target, and a distinct identifier tag. In another preferred embodiment, a tagged molecule generated by trimming includes an identifier tag and no copy or complement of the target. Tagged molecules may contain additional sequences, labels, chemical modifications, and other features selected by one of skill in the art for a particular embodiment. Tagged molecules interact with the universal detector, and the identifier tag in the tagged molecule hybridizes to complementary detection probes on the universal detector. It is the identifier tag that contains information content sufficient to indicate the presence of its corresponding target in a sample.

In a preferred embodiment, double-stranded amplification products can be trimmed, generating tagged molecules that can hybridize to a universal detector. Preferably, a nicking endonuclease is used to cut at sites flanking the tag sequence, where the nicking endonuclease cleaves only one strand of DNA of a double-stranded DNA substrate. The endonuclease recognition sequences are arranged in a dyad symmetric way around the tag sequence. For example, for *N.Bst*NB I, the recognition sequence GAGTC is placed four nucleotides 5' (upstream) of the beginning of the tag sequence on each strand. Such a trimming operation will release a tagged molecule containing an identifier tag, where the tagged molecule has "sticky ends" generated by one or more nicking endonucleases. Nicking endonucleases suitable for use in this embodiment include but are not limited to N.*Bst* NBI, N.*Bbv* CIA, N.*Alw* I, N.*Bbv* CIB (New England Biolabs, Beverly, MA). Advantageously, tagged molecules with sticky ends can hybridize to a surface, preferably the universal detector, more preferably an electrode surface. Tagged molecules, preferably hybridized to a surface, can undergo linear polymerization, providing a satisfactory level of hybridization to a surface, where any linear polymers that form by polymerization are also hybridized to a surface. In a preferred embodiment, oligonucleotides immobilized on a surface are biotinylated at their 3' end, as the sticky ends for DNA cut with N.*Bst* NBI and N.*Alw* I have 5' overhangs. Other enzymes such as N.*Bbv* CIB generate sticky ends with 3' overhangs, although the sequence need to create a nick cleavage site is more restricted than N.*Bst* NBI and N.*Alw.* Advantageously, surface hybridization of tagged molecules with sticky end enhanced by using detection probes that form a hairpin helix with biotin in the loop, providing a helix end for the tagged molecule to stack upon.

### C.3. Rolling circle transcription to generate tagged RNA molecules; trimming RNA molecules

Tagged RNA products suitable for use in the universal tag assay of the present invention can be generated by transcription of RC probes using methods known in the art, for example as disclosed by Kool (U.S. Patent Nos. 6,096,880 and 6,368,802). RNA synthesis by transcription of a RC probe (DNA) does not require a polymerization primer, although one may be used if desired. RNA synthesis by transcription of an RC probe does not require an RNA polymerase promoter sequence in the probe, although a RNA polymerase promoter sequence can be incorporated into the RC probe if desired. If an RC probe has no RNA polymerase promoter, transcription can be initiated at any location on the RC probe. If an RC probe has an RNA polymerase promoter, initiation of transcription is determined by the location of the promoter. Suitable RNA polymerases include but are not limited to T7, R4, T3, *E. coli* RNA polymerase, SP6 RNA polymerase, RCA polymerase II and III, or closely homologous mutants.

One aspect of the present disclosure provides an RC probe constructed such that it not only contains a copy or complement of target nucleotide sequence and a complement of an identifier tag sequence for that target nucleotide sequence, but also contains a sequence that encodes at least one biologically active RNA sequence, preferably a catalytic RNA sequence. In one embodiment, the RNA product generated by RC transcription preferably encodes a ribozyme and its cleavage site. Ribozymes suitable for use with the present invention include but are not limited to hairpin ribozymes, hammerhead-motif ribozymes, and hepatitis delta catalytic RNAs.

Hammerhead-motif catalytic RNAs can readily be adapted to cleave varied RNA sequences (Uhlenbeck, 1987, Nature 328:596-600; Haseloff et al., 1988, Nature 344:585-591; Symons, 1992, Ann Rev Biochem 61:641-671; Long et al., 1993, FASEB J, 7:25-30 by altering the sequence of the noncatalytic, substrate-binding domain of the RNA encoded by the RC probe that serves as a circular DNA template. Such modifications to the sequence of the substrate-binding domain are easily made during synthesis of the RC probe, thereby permitting the method of the invention to produce any desired diagnostically or biologically useful RNA. Monomeric catalytic RNAs can act not only in *cis* fashion (intramolecularly) but also in *trans* to cleave other target RNAs (Reddy et al., U.S. Patent No. 5,246,921; Cech et al., U.S. Patent Nos. 4,987,071, 5,354,855, 5,093,246). Catalytic RNAs produced by the invention include RNAs possessing any desired enzymatic activity, including but not limited to endo- or exo-nuclease activity, polymerase activity, ligase activity, or phosphorylase/dephosphorylase activity.

In accordance with the methods disclosed herein, the present invention provides multiple copies of a short, sequence-defined RNA oligonucleotide (oligoribonucleotide) tagged molecules formed by cleavage of the RNA product of RC transcription, where the RNA product contains repeating unit copies of the RC probe. In one embodiment, one autolytic site is present in the RC probe, such that cleavage of the transcipt generates tagged RNA molecules containing target sequence and an identifier tag. In another embodiment, more than one autolytic site is present in the RC probe and the identifier tag sequence is flanked by autolytic sites, such that cleavage of the transcript generates tagged RNA molecules containing identifier tag without target sequence. In a preferred embodiment, cleavage is autolytic, as where the monomeric units contain self cleaving ribozymes.

During transcription, the repeating RNAs may self cleave, producing tagged molecules of monomer length, (*i.e*., they are cleaved to produce oligonucleotides containing only one copy of the desired sequence) after a sufficient length of time has elapsed. In accordance with the present invention, a monomer may contain a copy or complement of the target nucleotide sequence and the identifier tag for that target, or a monomer may contain the identifier tag without target nucleotide sequence. Typically the monomers are linear, but they may be cyclic, for example when the monomer contains a hairpin-type ribozyme capable of intramolecular ligation. The resulting monomeric tagged molecules may include catalytically active ribozymes which can sequence-specifically cleave RNA targets in *trans.* As an example, a self cleaving multimer would result from inclusion of the hammerhead sequence (Forster et al., Cold Spring Harbor Symp Quant Biol, 52, 249 (1987)) in the RNA oligomer.

Cleavage of a concatemeric RNA product can also be accomplished chemically or enzymatically, as by contact with a second molecule possessing site-specific endonuclease enzymatic activity. The second molecule can be, for example, a protein or a ribozyme acting *in trans* to cleave a site located on a different nucleic acid. For example, an RNA multimer could also be cleaved at any sequence by using a hammerhead sequence used *in trans.* (Haseloff et al., 1988, Nature, 334:585). Another example of cleavage of an RNA multimer would be specific cleavage between G and A in the sequence 5 '-GAAA, which can be achieved by the addition of the oligomer 5'-UUU and Mn²⁺, following the method of Altman disclosed by Kazakov et al. (1992, Proc Natl Acad Sci USA, 89:7939-7943). RNA can also be cleaved using catalysts such as those disclosed by Chin (1992, J Am Chem Soc 114:9792), which have been attached to a DNA oligomer for sequence specificity. Alternatively, the enzyme RNase H can be used with addition of a DNA oligomer, or base-specific RNases can be used.

In another embodiment, self-cleaving monomeric ribozymes produced by RC transcription of circular DNA templates (RC probes) carry "stringency clamps" that may serve to increase their substrate sequence specificity, as disclosed by Kool et al. (U.S. Patent Nos. 6,096,880 and 6,368,802). The cleavage site in the concatemeric transcript is formed by intramolecular hybridization. Self-cleavage typically results in a monomeric tagged molecule in which the 5' and 3' ends are folded back onto the chain and duplexed in a hairpin configuration. To cleave in *cis,* binding of the substrate-binding sequences of the ribozyme monomer to the substrate must successfully compete with an intramolecular complement of the substrate-binding sequences. Stringency clamps advantageously reduce the susceptibility of the tagged molecules to degradation by various agents present in media, serum and the like.

### C.3. Target-dependent probe and primer binding to generate tagged molecules

Another aspect of the present disclosure provides tagged molecules generated using target-dependent processes that do not involve RC amplification, as illustrated in Figure 2. In accordance with this aspect, a tagged RNA molecule is generated by transcription of a DNA ligation product having sequence complementary to target nucleotide sequence flanked by sequence encoding an RNA polymerase promoter at the 3' end of the ligation product and sequence encoding an identifier tag at the 5' end of the ligation product. A tagged RNA molecule containing the identifier tag for a target nucleotide sequence will only be generated if primers complementary to the target nucleotide sequence successfully hybridized to the template target strand and were ligated. Target-dependent generation of tagged molecules includes but is not limited to the following steps: a) if necessary, obtaining single-stranded template having at least one target nucleotide sequence; b) contacting the template with a plurality of oligonucleotide primers, where at least one pair of ligation primers is designed to hybridize to at least one target nucleotide sequence on the template, such that the 5' end of one of the pair of ligation primers hybridizes adjacent to the 3' end of the other of the pair of ligation primer, and the primer having its 5' end hybridized to a portion of target nucleotide sequence additionally has sequence encoding an RNA polymerase promoter at its 3' end, and the primer having its 3' end hybridized to a portion of target nucleotide sequence additionally has sequence encoding an identifier tag for that target nucleotide sequence at its 5' end; c) incubating primers and template under conditions that promote hybridization to template and ligation of primers to form a ligation product; d) dissociating the ligation product from the template; e) repeating the hybridization and ligation steps as desired; f) recovering ligation products and incubating with RNA polymerase and auxiliary oligonucleotide to form RNA polymerase promoter. If desired, tagged RNA molecules generated by transcription may be recovered and then used in the universal tag assay. Optionally, tagged RNA molecules generated by transcription may be recovered and purified, and then used in the universal tag assay Alternately, the transcription reaction mixture may be utilized in the universal tag assay without any intervening recovery or clean-up steps.

Complements of ligation primers used in this embodiment should be designed to help prevent spurious signals. The complement of the tag-containing primer should be truncated such that it does not contain the complement of the tag sequence, and should be 3'-bloclced to prevent primer extension that might generate spurious copies of the tag.

This method may be used to identify variant or polymorphic sequences in a sample, including SNPs, splice variants, allelic form and mutants. Accordingly, a sample is incubated with a set of ligation primers including ligation primers having sequences complementary to variant sequences of the target nucleotide sequence, under conditions suitable for hybridization and ligation, wherein only those ligation primers complementary to the variant sequence present in the template will hybridize to the template and form at least one ligation product that further includes an RNA polymerase promoter and an identifier tag. In the embodiment wherein the identifier for the variant sequence is found at the 5' end of the ligation product, the primer whose 3' end hybridizes to a portion of target nucleotide sequence will be designed to discriminate which variant sequence is present. For example, if the variant is a SNP, the nucleotide at the 3' terminus of the primer whose 3' end hybridizes to a portion of target nucleotide sequence will be the nucleotide that discriminates which nucleotide is present in the variant being assayed. A plurality of targets, including a plurality of variant sequences, can be assayed simultaneously, as each target or variant has a distinct identifier tag that will only be present in a tagged molecule and bind to a complementary detection probe if the corresponding target was present in the sample being assayed.

Another aspect of the present disclosure provides a circularizable ligation product that can be used to generate an RC probe for rolling circle (RC) transcription. RC transcription generates tagged RNA molecules containing multiple repeating copies of the ligation product including the identifier tag. Tagged RNA molecules containing multiple repeating copies of sequence are suitable for use in the universal tag assay; alternately, these tagged molecules may be trimmed to generate smaller tagged RNA molecules containing an identifier tag.. RC transcription advantageously provides a convenient method for generating tagged RNA molecules in any quantity desired.

If desired, PCR can be used to amplify a region surrounding a target nucleotide sequence, where PCR products may provide a suitable substrate for the ligation and transcription reactions described above.

### D. Detection of variant sequences

In accordance with one aspect of the present disclosure ligation reactions are used to analyze variant or polymorphic sequences in the target nucleotide sequence, where such variant sequences include alleles of a locus, splice variants, or single nucleotide polymorphisms (SNPs). Advantageously, the high degree of specificity of ligation reactions permits discrimination among variant sequences to generate distinct tagged molecules corresponding to each distinct variant sequence, where the tagged molecules are easily detected using the universal tag assay. Figure 1 illustrates detecting a SNP in a sample, including the use of RC methods to generate tagged molecules in accordance with the present invention.

In accordance with one aspect of the present disclosure ligation reactions are used to identify variant or polymorphic sequences of the target nucleotide sequence present in a sample. Preferably, the variant sequence is a single nucleotide polymorphism SNP. Alternately, the variant sequence represents mutant or allelic forms of a target nucleotide sequence. In a preferred embodiment, the amplification step is carried out using a plurality of linear RC probes having sequences complementary to variant sequences of the target nucleotide sequence, wherein each linear RC probe is complementary to a single variant sequence and contains the complement of the identifier tag for that variant sequence. A sample is incubated with this plurality of linear RC probes under conditions suitable for hybridization and ligation of RC probes, such that only those RC probes complementary to the variant sequence(s) present in the sample will hybridize to the variant sequence in a target-dependent manner and be ligated to form a circularized RC probe suitable for RC amplification or RC transcription to generate tagged molecules suitable for use in the universal tag assay.

A plurality of variant sequences of the same or different target nucleotide sequences may be detected in a single reaction using a plurality of linear RC probes as described above, wherein each linear RC probe includes sequence complementary to a single variant sequence and a complement of the identifier tag for that variant sequence. Any variant sequence that is recognized by its corresponding RC probe and then amplified or transcribed by RC methods will be identified by its distinct identifier tag using the universal tag assay of the present invention.

Alternately, ligation reactions in a pre-amplification step are used to identify variant or polymorphic sequences of the target nucleotide sequence present in a sample. In a preferred embodiment, a set of ligation primers used in a pre-amplification step includes ligation primers having sequences complementary to variant sequences of the target nucleotide sequence. A sample is incubated with ligation primers having sequences complementary to variant sequences under conditions suitable for hybridization and ligation, wherein only those ligation primers complementary to variant sequences present in the target strand template will hybridize to the template and form at least one ligation product having sequence complementary to the variant target nucleotide sequence present in the template. In another preferred embodiment, the set of ligation primers includes primers having exogenous sequence such that the ligation product complementary to the variant sequence further includes exogenous nucleotide sequence at its 3' and 5' ends that is complementary to backbone sequence flanking a copy of target nucleotide sequence in an RC probe. The ligation products can be mixed with linear RC probes for target-dependent binding and ligation of the probes. Alternately, the ligation probes can be mixed with circular RC probes. The ligation products bound to RC probes can serve as polymerization primers for amplification of RC probes having complementary variant sequence.

A plurality of variant sequences of the same or different target nucleotide sequences may be detected in a single reaction using a plurality of ligation primers as described above, wherein ligation primers having sequence complementary to each variant sequence will produce a ligation product complementary to that variant sequence. Ligation products having sequence complementary to variant sequences will be amplified using RC probes having complementary variant sequence and a complement of the distinct identifier tag for that variant sequence, generating tagged molecules suitable for using in the universal tag assay

### E. Identification of organisms

Another aspect of the present disclosure is directed to methods for identifying an organism or individual by detecting one or more target nucleotide sequences chosen to serve as distinguishing features for the organism or individual. Each target nucleotide sequence is detected using the universal tag assay including but not limited to the following steps: a) obtaining template having at least one target nucleotide sequence; b) carrying out target-dependent manipulations to produce at least one tagged molecule containing at least one identifier tag for that target nucleotide sequence; c) incubating at least one tagged molecule with a universal detector having detection probes coupled to a detection means; and d) measuring hybridization of identifier tags to complementary detection probes. Hybridization of an identifier tag to its complementary detection probe indicates that the sample being assayed contained the corresponding target nucleotide sequence chosen to serve as a distinguishing feature for the organism or individual.

In one embodiment, target nucleotide sequence is detected using tagged molecules generated by amplification of template containing target nucleotide sequence, including but not limited to the steps of: a) obtaining template having at least one target nucleotide sequence; b) amplifying the template to generate amplification products containing target nucleotide sequence and an identifier tag for that target nucleotide sequence; c) incubating tagged molecule with a universal detector having detection probes coupled to detection means; and d) detecting hybridization of distinct identifier tags to complementary detection probes. Optionally, the amplification product also contains exogenous nucleotide sequences including sequences involved in trimming of amplification products, such that amplification products can be trimmed to generate smaller tagged molecules suitable for use in the universal tag assay. In this embodiment, an organism or individual may be identified by detecting a tagged molecule that indicates that the sample being assayed contained the corresponding target nucleotide sequence chosen to serve as a distinguishing feature for the organism or individual from sample taken from the organism or individual.

It is understood that each identifier tag used in an application has a complementary detection probe in the universal detector. Thus, an organism or individual may be identified by the hybridization of an identifier tag to its complementary detection probe, which reliably indicates the presence of the corresponding target in a sample. In addition, an organism or individual may also be identified by the absence of hybridization of a distinct identifier tag to its complementary detection probe, which reliably indicates the absence of the corresponding target in a sample. Preferably, internal controls are included to increase the reliability of this method as described herein. In another embodiment, a multiplicity of individuals or organisms is identified by this method. Advantageously, the universal tag assay and methods disclosed herein can be used with any organism or individual without the need for custom design or manufacture of detectors.

### F. Additional Alternative Embodiments Using Universal Tags

In some embodiments of assays using universal tag sequences, amplification of the template is performed after the identifier tag hybridizes to the detection probe. For example, one embodiment relates to a method for detecting a target nucleotide sequence in a sample using a rolling circle (RC) probe that comprises an identifier tag sequence which has been selected as an identifier corresponding to a particular target nucleotide sequence. In some embodiments the RC probe can also comprise a sequence complementary to a signal tag. Whether or not the RC probe comprises a sequence complementary to a signal tag will in part depend on the method that is selected for detecting the binding of the identifier tag sequence to the detection probe.

The sample which is interrogated with the RC probe can contain any of the templates described above including, but not limited to, genomic DNA, cDNA, hnRNA, mRNA, or amplification products or nucleic acid copies of any of these molecules. The template molecules can comprise one or more target sequences or complements thereof. In some embodiments the sample comprises one or more variants of each target sequence such as alternative alleles of a gene.

In some embodiments of the present disclosure the RC probe is linear. Such linear RC probes comprise 3' and 5' ends that are each complementary to at least a portion of a template molecule. In such embodiments, the linear RC probe is incubated with the template such that the 3' end and the 5' end of the RC probe hybridize adjacently to contiguous complementary sequence on the template thus leaving an unsealed nick between the ends of the RC probe. The 3' end and the 5' end can then be ligated thereby forming a circular RC padlock probe.

The circular RC padlock probe having an identifier tag therein can be hybridized with a universal detector which comprises one or more detection probes. The detection probes can be of uniform sequence or can be of various sequence types. In some embodiments of the present invention an array of detection probes is used. Such arrays will comprise at least one detection probe that is complementary to at least a portion of the identifier tag that is present in the RC padlock probe. In some embodiments, the detection probes are coupled to a detection means as described herein.

Upon hybridization of the identifier tag of the RC padlock probe with the detection probe, polymerization can be initiated from the free 3' end of the detection probe. Suitable polymerases for 5' to 3' extension of the detection probe using the RC probe as a template can be determined by one skilled in the art based on the conditions under which the polymerization occurs. Extension of the detection probe using the RC probe as template can result in one or more copies of the RC probe being produced as a catenated strand (see Figures 3a-c). If the RC probe also includes a sequence complementary to a signal tag, the extension product of the detection probe will comprise one or more copies of the signal tag sequence depending on the number of copies of the RC probe that are generated.

As a result of the extension reaction, the detection probe is extended from the free 3' end so as to produce an extended detection probe sequence (extension product) which comprises one or multiple copies of a sequence complementary to the RC probe. In some embodiments of the present invention, the RC probe remains associated with the extended detection probe sequence (extension product), whereas in other embodiments, the RC probe is dissociated from the extended detection probe sequence (extension product) thus leaving a free single-stranded detection probe product which comprises one or multiple copies of a sequence complementary to the RC probe. The extended detection probe sequence can be detected by any of the detection methods described herein including, but not limited to, amperometry using ruthenium. Additionally, if signal tag is present in the nucleic acid sequence complementary to the RC probe can be detected by hybridization with an appropriate signal probe. In some embodiments, the signal probe can comprise one or more modifications that facilitate bind of antibodies conjugates. For example, the signal probe can be labeled with fluoroscein and then detected using an antifluoroscein antibody conjugated to and enzyme such as horseradish peroxidase or POD.

Other embodiments of the present disclosure relate to methods wherein the reactions resulting in the amplification of a template molecule are all performed in the presence of the universal detector. For example, some embodiments relate to a method for detecting a target nucleotide sequence in a sample, using a universal detector comprising one or more detection probes coupled to a detection means. The universal detector is contacted with a template molecule which comprises a target nucleotide sequence or complement thereof. The template is amplified using any of the methods described herein thereby generating at least one molecule comprising at least one identifier tag. In some embodiments, the identifier tag is selected so that the tag is an identifier for a particular target nucleotide sequence. The identifier tag that is generated during the amplification step hybridizes with a detector probe that is complementary to the identifier tag. Hybridization of an identifier tag to a complementary detection probe can be detected using any of the methods described herein including amperometry. Detection of hybridization between an identifier tag and a detector probe indicates the presence in the sample of the target nucleotide sequence corresponding to the identifier tag.

### EXAMPLES

### Example 1. RC padlock probe in linear form

A linear DNA molecule suitable for use as an RC padlock probe is designed to hybridize to a single nucleotide polymorphism in the p53 gene known as p53 SNP3. The RC probe (SEQ ID NO: 8) contains sequence complementary to the target nucleotide sequence for the wild-type variant of p53 SNP3. Included in the probe are the following: a tag sequence known as the Z' tag (or, Z' "zipcode") (SEQ ID NO: 3), a T7 RNA polymerase promoter (SEQ ID NO: 4), an *Eco* RI restriction endonuclease site (SEQ ID NO: 5), and a 3' nucleotide gap (SEQ ID NO: 6) to aid T7 transcription. Sequence complementary to p53 SNP3 target nucleotide sequence is located on the 3' end (SEQ ID NO: 2) and 5' end (SEQ ID NO: 1) of SEQ ID NO: 8, with a 24-nucleotide sequence on the 5' end and a 13-nucleotide sequence on the 3' end. The "backbone" sequence of the RC probe containing non-target-complementary sequences is compared against the human genome and no comparable matches are found between the padlock probe backbone and sequences in the human genome.

RC probe for p53 SNP3, using Z' tag
5'end: GCACCTCAAAGCTGTTCCGTCCCA (SEQ ID NO: 1)
   Tm = 65.2°C; 24-mer
3' end: CAGGCACAAACAC (SEQ ID NO: 2)
   Tm = 42.0°C; 13-mer
Z' tag: AGCTACTGGCAATCT (SEQ ID NO: 3)
T7 promoter: CCCTATAGTGAGTCGTATTA (SEQ ID NO: 4)
Eco RI site: GAATTC (SEQ ID NO: 5)
3-nucleotide gap, helps transcription of T7 polymerase: GAT (SEQ ID NO: 6)
RC primer: GATAGGAGTCACTTAAGATCG (SEQ ID NO: 7)
Entire RC probe: (SEQ ID NO: 8) where the features of the RC probe (SEQ ID NO: 8) are identified as follows:

### Example 2. Ruthenium detection of products bound to carbon ink electrodes

### Immobilization of detection probe on universal chips

*Immobilization of Streptavidin or NeutrAvidin on the chips:* NeutrAvidin is dissolved in 10 mM HEPES/10 mM LiCl, pH 7.4 buffer containing 25% isopropyl alcohol. The NeutrAvidin solution at concentrations of 40 to 4,000 nM is deposited on the surface of working electrodes on a universal chip and allowed to dry completely at room temperature. StabilCoat solution, a solution to stabilize biomolecules, is added to the working electrodes on the universal chip and allowed to incubate for 10 minutes. The Stabilicoat solution is aspirated from the universal chip surface and the chip is dried briefly.

### Binding of detection probe on Neutr Avidin immobilized universal chip:

A detection probe which is biotinylated at the 5' end and contains a tag sequence complementary to the tag sequence of an RCA product, is incubated with a NeutrAvidin immobilized electrode surface at room temperature for 30 minutes. The biotinylated DNA solution is removed and the chip is washed by immersion in 10 mM HEPES/10 mM LiCl, pH 7.4 buffer. The chip with immobilized detection probes can be coated with a thin layer of Stabilcoat for storage.

### Hybridization of target nucleic acids to detection probe immobilized on a universal array:

A nucleic acid target with a portion of its sequence-the tag sequence complementary to the detection probe is applied to a universal chip and allowed to hybridize to the detection probe at room temperature for 30 minutes. Hybridization conditions such as salt concentration, the pH, incubation time, and temperature can be varied by one of skill in the art to optimize binding.

### Electrochemical measurement of immobilized DNA target:

DNA immobilized on the universal chip is detected using square wave voltammetry. Other methods such as cyclic voltammetry, differential pulse voltammetry can also be used. Rutheniumhexaamine, Ru(NH₃)₆³⁺, is a preferred cationic redox reporter for the detection of immobilized DNA on a universal chip. Square wave voltammetry for the detection of Ru(NH₃)₆³⁺ associated with surface DNA target is performed as follows. A three-electrode system is used: a silver wire reference electrode, a platinum wire auxiliary electrode and universal chip comprising carbon ink working electrodes with captured detection probes which may be sequence complementary to tag sequence, or may be DNA target nucleotide sequence. The chip is immersed in aqueous buffer containing 5µM Ru(NH₃)₆³⁺, 10 mM Tris/10 mM NaCl, pH 7.4 in an electrochemical cell. Square wave voltammograms are recorded after scanning from 0 to -500 mV at conditions of 25 mV amplitude, 4 mV step potential and 15 Hz frequency. The parameters of the square wave voltammetry can be varied as would be clear to those of skill in the art.

### Example 3. Transcription obligation products.

Polymerase chain reaction (PCR) is performed on a target region containing a single nucleotide polymorphism in the p53 gene known as p53 SNP 3. Two primers are then hybridized to the PCR product. Primer A contains a target-specific region on the 5' end, a T7 promoter region on the 3' end and a 3' biotin label. Primer B contains a target-specific region on the 3' end and a tag sequence Z' on the 5' end. Primers and sample are incubated under conditions wherein allele specific ligation takes place, thereby linking the T7 promoter region and the tag sequence Z' and generating a ligation product. The ligation product is captured by incubating the mixture with particles coated with streptavidin, and subsequently isolating the particles and discarding the supernatant such that excess primer B is washed away with the supernatant. A promoter oligonucleotide is then added which hybridizes to the promoter sequence in the ligation product, producing a double-stranded site on which transcription is initiated with the addition of the RNA polymerase. Transcription of the ligation product produces multiple copies of the complement of the Z' tag sequence. These multiple copies of the Z' complement are then exposed to a universal chip for hybridization to the Z' tag (detection probe) on the chip. Hybridization of the tag sequence to a complementary detection probe is measured by electrochemical detection of Ru(III) complex bound electrostatically to phosphodiesters, as described above.

### Example 4. PCR products tethered to a bead.

A 560 base pair (bp) region of the p53 gene is amplified by PCR using a forward primer containing a biotin label on the 5' end. The resulting double-stranded 560 bp PCR product contains single nucleotide polymorphisms (SNPs) 1, 2, and 3. The double-stranded product is denatured and the bottom strand is washed away, resulting in several copies of the single-stranded 560 nucleotide region. The three relevant padlock probes (70.24, 70.31, and 70.33 containing tag sequences X', Y', and Z' respectively) are hybridized to the target sequences in the PCR product. Allele specific ligation is performed, ligating the padlock probes to the target regions. The PCR product with ligated padlock probes containing a 5' Biotin label is captured with Streptavidin beads and the excess padlock probe is washed away. Linear RC amplification is then performed simultaneously on the three padlocks, producing multiple length RCA products containing complements to the tag sequences X' (SNP 1), Y' (SNP 2), and Z' (SNP 3). These RC amplification products are exposed to a universal chip for hybridization to the tag sequences on the chip. The hybridization of the amplification product is read out by electrochemical detection of Ru(III) complex bound electrostatically to phosphodiesters.

### Example 5. On Chip extension of a detection probe and signal detection using an antibody

This Example demonstrates the detection of hybridization of an identifier tag to a detection probe by detecting a single-stranded nucleic acid extended from the free 3' end of a detection probe using an antifluoroscein antibody.

### Deposit of Detection Probe

An oligonucleotide having the sequence 5'- GTACGGATAACTACG - 3' (SEQ ID NO: 9), to be fixed to the suface of a universal chip for use as a detection probe, was dissolved to a final concentration of 6 µM in a buffer comprising 10 mM HEPS, 50 mM LiC1 and 0.4 mg/ml NeutraAvidin at pH 7.4. After 20 minutes the solution was adjusted to contain 12.5 % isopropanol and 6 µl of the solution was deposited onto a universal detector chip. The chip was dried at room temperature, coated with Stable coat for 20 minutes, and then dried in a vacuum. To remove any unbound detection probe, the chip was soaked at 37 °C in a solution of 10 mM HEPS, 200 mM LiC1 and 0.05% Tween 20 at pH 7.5 for 15 minutes then rinsed twice with a solution of 10 mM HEPS and 10 mM NaC1 at pH 7.4.

### Hybridization of Circular Probe to a Detection Probe Having a Sequence Complementary to the Identification Tag Present on an RC Probe

About 2.5 pM of circular RC probe 75.03 having the sequence 5' - GCCTGTCCAGGGATCTGCTCTTACCCTATAGTGAGTCGTATTACGTAGTTATCCGTACC AATACCTGTATTCCTT - 3' (SEQ ID NO: 10) was dissolved in a buffer of 10 mM HEPS, 1 M LiC1 and 0.05% Tween 20. The probe solution was transferred to a chip comprising the detection probe of SEQ ID NO: 9. Subsequently, the chip was preheated to 60 °C, incubated at 60 °C for five minutes and then cooled to room temperature for 30 minutes. After cooling, the chip was washed with a buffer of 10 mM HEPS, 233 mM LiC1 and 0.05% Tween 20 at pH 7.4 then rinsed with a buffer of 10 mM HEPS supplemented with 200 mM NaCl at pH 7.4. Both the wash and rinse steps were repeated. RCA Reaction to Extend the 3' End of the Detection Probe Using the RC Probe as Template

RCA was performed by adding to the chip dNTPs (1.5 µM final concentration) and φ29 polymerase (New England Biolabs) dissolved in φ29 polymerase buffer supplemented with 100 mM KCl. The RCA extension reaction was incubated for 1 hour at 37 °C for one hour then at room temperature for 30 minutes. The chip was washed twice with a buffer comprising 10 mM HEPS, 233 mM LiCl and 0.05% Tween 20 at pH 7.5 then rinsed with 10 mM Tris containing 200 mM NaCl. The chip was then soaked in this solution.

### Hybridization of the Signal Probe to the Extended RCA Product

Chips were incubated with 0.25 µM of a fluoroscein containing signal probe T7-F2 having the sequence 5' - CCTATAGTGAGTCGT - 3' (SEQ ID NO: 11) in a hybridization buffer comprising 10 mM Tris, 1 M NaCl, 0.05% Tween 20 and 0.05% bovine serum albumin (BSA). Hybridization occurred at 37 °C for 10 minutes then at room temperature for an additional 30 minutes. Following the hybridization, chips were washed with PBS comprising 0.05% Tween 20.

### Detection of Signal Probe using an Antifluoroscein Antibody

Chips were incubated for 20 minutes at room temperature with a 1:200 dilution of antifluoroscein antibody conjugated to POD in PBS buffer comprising 0.5% casein and 0.05% Tween 20. The chips were then washed with PBS containing 0.05% Tween 20 and signal was detected using K-blue TMB. Figures 4a and 4b show that signal (current) was only generated using RC probe complementary to a detection probe (signal). When the experiment was performed using a no RC probe control (NTC) or an RC probe non-complementary to detection probe (NCC), no current was generated.

### Example 6. On chip extension of a detection probe and signal detection using amperometry

This Example demonstrates the detection of hybridization of an identifier tag to a detection probe by detecting a single-stranded nucleic acid extended from the free 3' end of a detection probe using amperometry.

For this experiment, RC probe 75.03 (SEQ ID NO: 10) was used. RC probe 75.03 has 5' and 3' ends that are each complementary to a portion of a region of human genomic DNA (corresponding the human factor V sequence) such that, when incubated with such DNA or copy thereof, the 5' end hybridizes adjacent to the 3' end leaving only an unsealed nick between the two ends. Human genomic DNA comprising sequence complementary to the 5' and 3' ends of RC probe 75.03 was amplified using PCR. A portion of the PCR product was mixed with ligase buffer, Tth ligase and approximately 50 nM of linear RC probe 75.03. Ligation of the probe ends was performed by incubating the mixture for forty cycles at alternating temperatures between 95 °C for 30 seconds and 60 °C for 1 minute.

A sample of the ligation mixture was withdrawn and LiCl was added to a final concentration of 1 M. The reaction mixture was then added to a universal detector chip having fixed to its surface detector probes complementary to the identifier tag present on the circularized RC probe. The probe was hybridized to the detector probe by heating the chip to 60 °C for 5 minutes then cooling to room temperature for 20 minutes. The chip was then washed three times with a ruthenium detection solution containing 5 µM Ru(NH₃)₆³⁺, 10 mM Tris and 10 mM NaCl at pH 7.4.

Subsequent to hybridization, RCA was performed by adding to the chip a solution comprising φ29 polymerase buffer, dNTPs, BSA, pyrophosphatase and φ29 polymerase (New England Biolabs). The RCA extension reaction was incubated for 1 hour at 37 °C then at room temperature for 30 minutes. After this incubation, the chip was washed three times with a ruthenium detection solution containing 5 µM Ru(NH₃)₆³⁺, 10 mM Tris and 10 mM NaCl at pH 7.4. Binding of ruthenium to DNA was measured using amperometry.

## Claims

1. A method for detecting a target nucleotide sequence in a sample, said method comprising the steps of:
a) generating at least one tagged molecule comprising at least one identifier tag selected as an identifier for said target nucleotide sequence, wherein said identifier tag is generated only when said target nucleotide sequence corresponding to said identifier tag is present in said sample;
b) incubating said at least one tagged molecule with a universal detector having at least one detection probe complementary to said identifier tag, said universal detector comprising an array of detection probes coupled to electrochemical detection means, said array comprising electrodes attached to a substrate; and
c) detecting hybridization of any said identifier tag to any said detection probe complementary to said identifier tag, wherein said hybridization of any said identifier tag to any said detection probe complementary to said identifier tag indicates said target nucleotide sequence corresponding to said identifier tag is present in said sample.

2. The method of Claim 1, wherein said at least one tagged molecule further comprises a copy or complement of said target nucleotide sequence.

3. The method of Claim 2, wherein said detection probes coupled to a detection means are attached to a surface, film, or particle.

4. The method of Claim 6, wherein said detection probes are attached to said surface, film, or particle by covalent bonds, ionic bonds, electrostatic interactions, or adsorption.

5. The method of Claim 1, wherein said electrodes are gold or carbon.

6. The method of Claim 11, wherein said electrodes are gold.

7. The method of Claim 1, wherein the step of detecting hybridization comprises measuring hybridization of any said identifier tag to any said detection probe complementary to said identifier tag using a transition metal complex capable of oxidizing at least one oxidizable base in an oxidation-reduction reaction under conditions that cause an oxidation-reduction reaction between said transition metal complex and said oxidizable base, wherein said detection probe and/or said identifier tag comprise at least one oxidizable base.

8. The method of Claim 7, wherein said step of detecting hybridization comprises ruthenium amperometry.

9. The method of Claim 1, wherein said electrodes are coated with protein which can be bound by oligonucleotides derivatized with a moiety that binds said protein that coats said electrode.

10. The method of Claim 9, wherein said electrodes are coated with avidin such that said electrodes can be bound by biotin-labelled oligonucleotides.

11. The method of any preceding claim, wherein said step of generating at least one tagged molecule comprising at least one identifier tag selected as an identifier for said target nucleotide sequence comprises amplifying a template present in said sample, wherein said template comprises said target nucleotide sequence or complement thereof.

12. The method of any preceding claim for detecting a plurality of target nucleotide sequences in a sample, wherein each target nucleotide sequence in said plurality of target nucleotide sequences has a distinct identifier tag, such that hybridization of each said distinct identifier tag to a complementary detection probe indicates the presence of the corresponding target nucleotide sequence.

13. The method of any preceding claim, wherein said at least one tagged molecule comprises exogenous nucleotide sequence not found in said identifier tag or said target nucleotide sequence.

14. The method of Claim 13, wherein said exogenous nucleotide sequence is sequence involved in trimming tagged molecules and said method further comprises trimming said at least one tagged molecule to generate at least one smaller tagged molecule.

15. The method of Claim 11, wherein said amplifying said template comprises rolling circle (RC) amplification comprising the steps of:
a) providing a rolling circle (RCA) probe comprising sequence complementary to said template comprising target nucleotide sequence or complement thereof, and further comprising sequence complementary to said identifier tag selected as an identifier for said target nucleotide sequence;
b) incubating said RC probe with said template under conditions such that said RC probe will hybridize to complementary sequence on said template;
c) providing polymerase enzyme under conditions such that said polymerase replicates said RC probe, generating at least one amplification product comprising a tagged molecule having repeating copies of said identifier tag and said target nucleotide sequence or complement thereof;
d) incubating said amplification product with a universal detector comprising detection probes coupled to a detection means, said detection probes comprising at least one detection probe complementary to said identifier tag; and
e) detecting hybridization of any said identifier tag in said amplification product to any said detection probe complementary to said identifier tag;
wherein hybridization of any said identifier tag to any said complementary detection probe indicates the presence of the corresponding target nucleotide sequence in the sample being assayed.

16. The method of Claim 15, wherein said RC probe provided in step a) is a circular RC probe.

17. The method of Claim 15, wherein said RC probe provided in step a) is linear, such that step b) further comprises incubating said RC probe with said template under conditions such that the 3' end of said RC probe and the 5' end of said RC probe will hybridize adjacently to contiguous complementary sequence on said template, and said 3' end and said 5' end are ligated to form a circular RC padlock probe.

18. The method of Claim 11 or any of Claims 15 to 17, wherein said template is DNA or RNA.

19. The method of any of Claims 15 to 17, wherein said at least one amplification product further comprises exogenous sequence not found in said identifier tag or said template comprising target nucleotide sequence or complement thereof.

20. The method of Claim 19, wherein said exogenous sequence is involved in primer binding to said amplification product, forming polymerase promoters on said amplification product, or trimming said amplification product.

21. The method of Claim 20, wherein said exogenous sequence involved in trimming said amplification product comprises self-complementary sequences that form hairpin structures.

22. The method of Claim 21, wherein said self complementary sequences that form hairpin structures comprise at least one restriction enzyme recognition site for a restriction enzyme involved in said trimming.

23. The method of Claim 20, wherein said at least one exogenous nucleotide sequence comprises sequences that form at least one restriction enzyme recognition site for a restriction enzyme involved in said trimming upon addition of at least one auxiliary oligonucleotide.

24. The method of Claim 11, wherein said amplifying said template comprises ligation-mediated amplification comprising:
a) providing at least one pair of ligation pimers comprising a first ligation primer having a 3' portion of sequence complementary to a portion of said template comprising said target nucleotide sequence or complement thereof, and a second ligation primer having a 5' portion of sequence complementary to a contiguous portion of said template comprising target nucleotide sequence or complement thereof, wherein at least one ligation primer further comprises an identifier tag selected as an identifier for said target nucleotide sequence;
b) incubating said at least one pair of ligation primers with said template under conditions such that said first and second ligation primers will hybridize to said template, said 3' end of said first ligation primer hybridizing adjacently to said 5' end of said second ligation primer;
c) ligating said 3' end of said first ligation primer to said 5' end of said second ligation primer, thereby generating a ligation product comprising a tagged molecule, wherein said tagged molecule comprises a copy or complement of said target nucleotide sequence and further comprises at least one identifier tag corresponding to said target nucleotide sequence;
d) contacting said ligation product with a universal detector comprising detection probes coupled to a detection means, said detection probes comprising at least one detection probe complementary to said identifier tag; and
e) detecting hybridization of any said identifier tag in said ligation product to any said detection probe complementary to said identifier tag;
wherein hybridization of any said identifier tag to any said complementary detection probe indicates the presence of the corresponding target nucleotide sequence in the sample being assayed.

25. The method of Claim 24, wherein said template comprises target nucleotide sequence, such that said first ligation primer comprises sequence complementary to a portion of said target nucleotide sequence and said second ligation primer comprises sequence complementary a contiguous portion of said target nucleotide sequence, with the result that said first ligation primer and said second ligation primer hybridize to contiguous portions of template and are ligated, generating a ligation product comprising an identifier tag and sequence complementary to said target nucleotide sequence.

26. The method of Claim 24, wherein said template comprises complement of target nucleotide sequence, such that said first ligation primer comprises a portion of said target nucleotide sequence and said second ligation primer comprises a contiguous portion of said target nucleotide sequence, with the result that said first ligation primer and said second ligation primer hybridize to contiguous portions of template and are ligated, generating a ligation product comprising an identifier tag and said target nucleotide sequence.

27. The method of any of Claims 24 to 26, wherein said ligating said 3' end of said first ligation primer to said 5' end of said second ligation primer is carried out by enzymatic means.

28. The method of Claim 27, wherein said enzymatic means is ligase.

29. The method of any of Claims 24 to 26, wherein said ligating said 3' end of said first ligation primer to said 5' end of said second ligation primer is carried out by non-enzymatic means.

30. The method of any of Claims 24 to 26, wherein said incubating and ligating steps are repeated using temperature cycling for amplification of said target nucleotide sequence.

31. The method of any of Claims 24 to 26, for detecting a plurality of target nucleotide sequences, comprising providing a plurality of pairs of ligation primers wherein at least one said pair of ligation primers of said plurality of pairs is complementary to each target nucleotide sequence of said plurality of target nucleotide sequences, and further wherein at least one ligation primer of each said pair comprises an identifier tag selected to serve as an identifier for said target nucleotide sequence.

32. The method of any of Claims 24 to 26, wherein said method comprises detecting at least one variant sequence of said target nucleotide sequence.

33. The method of Claim 32, wherein said at least one variant sequence is a single nucleotide polymorphism (SNP), an allelic variant, or a splice variant.

34. The method of Claim 33, wherein said at least one variant sequence is a SNP.

35. A composition for detecting targets in a sample using a universal tag assay comprising:
a) a set of minimally cross-hybridizing tags and probes selected such that at least one tag will serve as an identifier tag for a target in a sample being assayed and each said identifier tag has at least one complementary detection probe in said set;
b) at least one tagged molecule comprising at least one said identifier tag, wherein said identifier tag is generated only when said target corresponding to said identifier tag is present in said sample being assayed; and
c) a universal detector comprising at least one said detection probe coupled to an electrochemical detection means such that hybridization of said any identifier tag to any said complementary detection probe can be measured wherein said universal detector comprises an array of detection probes coupled to detection means, said array comprising electrodes attached to a substrate; wherein measuring hybridization of any said identifier tag to any said complementary detection probe indicates that said target corresponding to said identifier tag is present in said sample being assayed.

36. The composition of Claim 35, wherein said at least one tagged molecule comprises the identifier tag for a target and further comprises a copy or complement of said target.

37. The composition of Claim 35, wherein said at least one tagged molecule comprises the identifier tag molecule for a target and does not contain a copy or complement of said target.

38. The composition of Claim 35, wherein said electrodes are gold or carbon.

39. The composition of Claim 36, wherein said electrodes are gold.

40. The composition of Claim 35, wherein said electrodes are coated with protein which can be bound by oligonucleotides derivatized with a moiety that binds said protein that coats said electrode.

41. The composition of Claim 35, wherein said electrodes are coated with avidin such that said electrodes can be bound by biotin-labelled oligonucleotides.

42. The composition of Claim 35, wherein said set of minimally cross-hybridizing tags and probes is selected such that all probe/tag duplexes have the same or similar melting temperature and stacking energy.

## Patentansprüche

1. Verfahren zum Nachweisen einer Zielnukleotidsequenz in einer Probe, wobei das genannte Verfahren die folgenden Schritte beinhaltet:
a) Erzeugen von wenigstens einem markierten Molekül, das wenigstens eine Kennmarke umfasst, die als Kennung für die genannte Zielnukleotidsequenz ausgewählt ist, wobei die genannte Kennmarke nur dann erzeugt wird, wenn die genannte Zielnukleotidsequenz, die der genannten Kennmarke entspricht, in der genannten Probe vorliegt;
b) Inkubieren des genannten wenigstens einen markierten Moleküls mit einem Universaldetektor mit wenigstens einer Nachweissonde, die zu der genannten Kennmarke komplementär ist, wobei der genannte Universaldetektor eine Anordnung von Nachweissonden umfasst, die mit elektrochemischen Nachweismitteln gekoppelt sind, wobei die genannte Anordnung an einem Substrat angebrachte Elektroden umfasst; und
c) Nachweisen einer Hybridisierung einer genannten Kennmarke mit einer genannten Nachweissonde, die zu der genannten Kennmarke komplementär ist, wobei die genannte Hybridisierung einer genannten Kennmarke mit einer genannten Nachweissonde, die zu der genannten Kennmarke komplementär ist, anzeigt, dass die genannte Zielnukleotidsequenz, die der genannten Kennmarke entspricht, in der genannten Probe vorliegt.

2. Verfahren nach Anspruch 1, wobei das genannte wenigstens eine markierte Molekül ferner eine Kopie oder ein Komplement der genannten Zielnukleotidsequenz umfasst.

3. Verfahren nach Anspruch 2, wobei die genannten mit einem Nachweismittel gekoppelten Nachweissonden auf einer Oberfläche, einer Folie oder einem Partikel angelagert sind.

4. Verfahren nach Anspruch 6, wobei die genannten Nachweissonden an der genannten Oberfläche, der genannten Folie oder dem genannten Partikel durch kovalente Bindungen, ionische Bindungen, elektrostatische Interaktionen oder Adsorption angelagert sind.

5. Verfahren nach Anspruch 1, wobei die genannten Elektroden aus Gold oder Kohlenstoff sind.

6. Verfahren nach Anspruch 11, wobei die genannten Elektroden aus Gold sind.

7. Verfahren nach Anspruch 1, wobei der Schritt des Nachweisens einer Hybridisierung das Messen einer Hybridisierung einer genannten Kennmarke mit einer genannten, zu der genannten Kennmarke komplementären Nachweissonde mit einem Übergangsmetallkomplex beinhaltet, der wenigstens eine oxidierbare Base in einer Oxidationsreduktionsreaktion unter Bedingungen oxidieren kann, die eine Oxidationsreduktionsreaktion zwischen dem genannten Übergangsmetallkomplex und der genannten oxidierbaren Base bewirken, wobei die genannte Nachweissonde und/oder die genannte Kennmarke wenigstens eine oxidierbare Base umfasst/umfassen.

8. Verfahren nach Anspruch 7, wobei der genannte Schritt des Nachweisens einer Hybridisierung Ruthenium-Amperometrie beinhaltet.

9. Verfahren nach Anspruch 1, wobei die genannten Elektroden mit Protein beschichtet sind, das durch Oligonukleotide gebunden werden kann, die mit einem Anteil derivatisiert sind, der das genannte Protein bildet, mit dem die genannte Elektrode beschichtet ist.

10. Verfahren nach Anspruch 9, wobei die genannten Elektroden mit Avidin beschichtet sind, so dass die genannten Elektroden durch Biotin-etikettierte Oligonukleotide gebunden werden können.

11. Verfahren nach einem der vorherigen Ansprüche, wobei der genannte Schritt des Erzeugens von wenigstens einem markierten Molekül, das wenigstens eine Kennmarke umfasst, die als Kennung für die genannte Zielnukleotidsequenz ausgewählt ist, das Amplifizieren eines in der genannten Probe vorliegenden Template beinhaltet, wobei das genannte Template die genannte Zielnukleotidsequenz oder ein Komplement davon umfasst.

12. Verfahren nach einem der vorherigen Ansprüche zum Nachweisen mehrerer Zielnukleotidsequenzen in einer Probe, wobei jede Zielnukleotidsequenz der mehreren Zielnukleotidsequenzen eine eigene Kennmarke hat, so dass eine Hybridisierung jeder genannten eigenen Kennmarke mit einer komplementären Nachweissonde die Anwesenheit der entsprechenden Zielnukleotidsequenz anzeigt.

13. Verfahren nach einem der vorherigen Ansprüche, wobei das genannte wenigstens eine markierte Molekül eine exogene Nukleotidsequenz umfasst, die in der genannten Kennmarke oder der genannten Zielnukleotidsequenz nicht zu finden ist.

14. Verfahren nach Anspruch 13, wobei die genannte exogene Nukleotidsequenz die Sequenz ist, die am Beschneiden von markierten Molekülen beteiligt ist, und wobei das genannte Verfahren ferner das Beschneiden des genannten wenigstens einen markierten Moleküls beinhaltet, um wenigstens ein kleineres markiertes Molekül zu erzeugen.

15. Verfahren nach Anspruch 11, wobei das genannte Amplifizieren des genannten Template Rollkreis-(RC)-Amplifikation beinhaltet, die die folgenden Schritte beinhaltet:
a) Bereitstellen einer Rollkreis-(RC)-Sonde, die Sequenz umfasst, die zu dem genannten Template komplementär ist, die Zielnukleotidsequenz oder ein Komplement davon umfasst, und die ferner Sequenz umfasst, die zu der genannten Kennmarke komplementär ist, die als Kennung für die genannte Zielnukleotidsequenz ausgewählt ist;
b) Inkubieren der genannten RC-Sonde mit dem genannten Template unter solchen Bedingungen, dass die genannte RC-Sonde mit einer komplementären Sequenz auf dem genannten Template hybridisiert;
c) Bereitstellen eines Polymeraseenzyms unter solchen Bedingungen, dass die genannte Polymerase die genannte RC-Sonde repliziert und wenigstens ein Amplifikationsprodukt erzeugt, das ein markiertes Molekül mit sich wiederholenden Kopien der genannten Kennmarke und die genannte Zielnukleotidsequenz oder ein Komplement davon umfasst;
d) Inkubieren des genannten Amplifikationsprodukts mit einem Universaldetektor, der Nachweissonden umfasst, die mit einem Nachweismittel gekoppelt sind, wobei die genannten Nachweissonden wenigstens eine Nachweissonde umfassen, die zu der genannten Kennmarke komplementär ist; und
e) Nachweisen einer Hybridisierung einer genannten Kennmarke in dem genannten Amplifikationsprodukt mit einer genannten Nachweissonde, die zu der genannten Kennmarke komplementär ist;
wobei eine Hybridisierung einer genannten Kennmarke mit einer genannten komplementären Nachweissonde die Anwesenheit der entsprechenden Zielnukleotidsequenz in der untersuchten Probe anzeigt.

16. Verfahren nach Anspruch 15, wobei die in Schritt a) bereitgestellte genannte RC-Sonde eine kreisf8nnige RC-Sonde ist.

17. Verfahren nach Anspruch 15, wobei die in Schritt a) bereitgestellte genannte RC-Sonde linear ist, so dass Schritt b) ferner das Inkubieren der genannten RC-Sonde mit dem genannten Template unter solchen Bedingungen beinhaltet, dass das 3'-Ende der genannten RC-Sonde und das 5'-Ende der genannten RC-Sonde neben aneinander angrenzender komplementärer Sequenz auf dem genannten Template hybridisiert und das genannte 3'-Ende und das genannte 5'-Ende zu einer kreisförmigen RC-Vorhängeschloss-Sonde ligiert werden.

18. Verfahren nach Anspruch 11 oder einem der Ansprüche 15 bis 17, wobei das genannte Template DNA oder RNA ist.

19. Verfahren nach einem der Ansprüche 15 bis 17, wobei das genannte wenigstens eine Amplifikationsprodukt ferner exogene Sequenz umfasst, die in der genannten Kennmarke oder dem genannten Template nicht zu finden ist, das eine Zielnukleotidsequenz oder ein Komplement davon umfasst.

20. Verfahren nach Anspruch 19, wobei die genannte exogene Sequenz an einer Primer-Bindung an das genannte Amplifikationsprodukt, dem Bilden von Polymerasepromotoren an dem genannten Amplifikationsprodukt oder dem Beschneiden des genannten Amplifikationsprodukts beteiligt ist,

21. Verfahren nach Anspruch 20, wobei die am Beschneiden des genannten Amplifikationsprodukts beteiligte exogene Sequenz relbstkoinplementäre Sequenzen umfasst, die Haamadelstrukturen bilden,

22. Verfahren nach Anspruch 21, wobei die genannten selbstkomplementären Sequenzen, die Haamadelstrukturen bilden, wenigstens eine Restriktionsenzymerkennungsstelle für ein an der genannten Beschneidung beteiligtes Restriktionsenzym umfassen.

23. Verfahren nach Anspruch 20, wobei die genannte wenigstens eine exogene Nukleotidsequenz Sequenzen umfasst, die wenigstens eine Restriktionsenzymerkennungsstelle für ein an der genannten Beschneidung beteiligtes Restriktionsenzym nach Zugabe von wenigstens einem zusätzlichen Oligonukleotid bilden.

24. Verfahren nach Anspruch 11, wobei das genannte Amplifizieren des genannten Template eine ligationsvermittelte Amplifizierung beinhaltet, die Folgendes beinhaltet:
a) Bereitstellen von wenigstens einem Paar Ligationsprimer, das einen ersten Ligationsprimer mit einem 3'-Abschnitt einer Sequenz, die zu einem Abschnitt des genannten Template komplementär ist, das die genannte Zielnukleotidsequenz oder ein Komplement davon umfasst, und einen zweiten Ligationsprimer mit einem 5'-Abschnitt von Sequenz umfasst, die zu einem angrenzenden Teil des genannten Template komplementär ist, das Zielnukleotidsequenz oder ein Komplement davon umfasst, wobei wenigstens ein Ligationsprimer ferner eine Kennmarke umfasst, die als Kennung für die genannte Zielnukleotidsequenz ausgewählt ist;
b) Inkubieren des genannten wenigstens einen Ligationsprimerpaares mit dem genannten Template unter solchen Bedingungen, dass der genannte erste und zweite Ligationsprimer mit dem genannten Template hybridisieren, wobei das genannte 3'-Ende des genannten ersten Ligationsprimers neben dem genannten 5'-Ende des genannten zweiten Ligationsprimers hybridisiert;
c) Ligieren des genannten 3'-Endes des genannten ersten Ligationsprimers mit dem genannten 5'-Ende des genannten zweiten Ligationsprimers, um **dadurch** ein Ligationsprodukt zu erzeugen, das ein markieres Molekül umfasst, wobei das genannte markierte Molekül eine Kopie oder ein Komplement der genannten Zielnukleotidsequenz umfasst und ferner wenigstens eine Kennmarke umfasst, die der genannten Zielnukleotidsequenz entspricht;
d) Kontaktieren des genannten Ligationsprodukts mit einem Universaldetektor, der Nachweissonden umfasst, die mit einem Nachweismittel gekoppelt sind, wobei die genannten Nachweissonden wenigstens eine zu der genannten Kennmarke komplementäre Nachweissonde umfassen; und
e) Nachweisen einer Hybridisierung einer genannten Kennmarke in dem genannten Ligationsprodukt mit einer zu der genannten Kennmarke komplementären Nachweissonde;
wobei die Hybridisierung einer genannten Kennmarke mit einer komplementären Nachweissonde die Anwesenheit der entsprechenden Zielnukleotidsequenz in der untersuchten Probe anzeigt.

25. Verfahren nach Anspruch 24, wobei das genannte Template Zielnukleotidsequenz umfasst, so dass der genannte erste Ligationsprimer Sequenz umfasst, die zu einem Abschnitt der genannten Zielnukleotidsequenz komplementär ist, und der genannte zweite Ligationsprimer Sequenz umfasst, die zu einem angrenzenden Abschnitt der genannten Zielnukleotidsequenz komplementär ist, mit dem Ergebnis, dass der genannte erste Ligationsprimer und der genannte zweite Ligationsprimer mit angrenzenden Template-Abschnitten hybridisieren und ligiert werden und dabei ein Ligationsprodukt erzeugen, das eine zu der genannten Zielnukleotidsequenz komplementäre Kennmarke und Sequenz umfasst.

26. Verfahren nach Anspruch 24, wobei das genannte Template ein Komplement der Zielnukleotidsequenz umfasst, so dass der genannte erste Ligationsprimer einen Abschnitt der genannten Zielnukleotidsequenz umfasst und der genannte zweite Ligationsprimer einen angrenzenden Abschnitt der genannten Zielnukleotidsequenz umfasst, mit dem Ergebnis, dass der genannte erste Ligationsprimer und der genannte zweite Ligationsprimer mit angrenzenden Template-Abschnitten hybridisieren und ligiert werden und dabei ein Ligationsprodukt erzeugen, das eine Kennmarke und die genannte Zielnukleotidsequenz umfasst.

27. Verfahren nach einem der Ansprüche 24 bis 26, wobei das genannte Ligieren des genannten 3'-Endes des genannten ersten Ligationsprimers mit dem genannten 5'-Ende des genannten zweiten Ligationsprimers mit enzymatischen Mitteln erfolgt.

28. Verfahren nach Anspruch 27, wobei das genannte enzymatische Mittel Ligase ist.

29. Verfahren nach einem der Ansprüche 24 bis 26, wobei das genannte Ligieren des genannten 3'-Endes des genannten ersten Ligationsprimers mit dem genannten 5'-Ende des genannten zweiten Ligationsprimers mit nichtenzymatischen Mitteln erfolgt.

30. Verfahren nach einem der Ansprüche 24 bis 26, wobei der genannte Inkubier- und der genannte Ligationsschritt mit Temperatur-Cycling zum Amplifizieren der genannten Zielnukleotidsequenz wiederholt werden.

31. Verfahren nach einem der Ansprüche 24 bis 26 zum Nachweisen mehrerer Zielnukleotidsequenzen, das das Bereitstellen mehrerer Paare von Ligationsprimern beinhaltet, wobei wenigstens ein genanntes Paar von Ligationsprimern aus der genannten Mehrzahl von Primern zu jeder Zielnukleotidsequenz der genannten Mehrzahl von Zielnukleotidsequenzen komplementär ist, und wobei ferner wenigstens ein Ligationsprimer jedes genannten Paares eine Kennmarke umfasst, die so gewählt ist, dass sie als Kennung für die genannte Zielnukleotidsequenz dient.

32. Verfahren nach einem der Ansprüche 24 bis 26, wobei das genannte Verfahren das Nachweisen von wenigstens einer Sequenzvariante der genannten Zielnukleotidsequenz beinhaltet.

33. Verfahren nach Anspruch 32, wobei die genannte wenigstens eine Sequenzvariante ein einzelner Nukleotidpolymorphismus (SNP), eine allele Variante oder eine Splice-Variante ist.

34. Verfahren nach Anspruch 33, wobei die genannte wenigstens eine Sequenzvariante ein SNP ist.

35. Zusammensetzung zum Nachweisen von Zielen in einer Probe unter Verwendung eines universellen Tag-Assay, die Folgendes umfasst:
a) einen Satz minimal kreuzhybridisierender Marker und Sonden, die so gewählt sind, dass wenigstens eine Marke als Kennmarke für ein Ziel in einer untersuchten Probe dient und jede genannte Kennmarke wenigstens eine komplementäre Nachweissonde in dem genannten Satz hat;
b) wobei wenigstens ein markiertes Molekül wenigstens eine genannte Kennmarke umfasst, wobei die genannte Kennmarke nur dann erzeugt wird, wenn das genannte Ziel, das der genannten Kennmarke entspricht, in der genannten untersuchten Probe vorhanden ist; und
c) einen Universaldetektor, der wenigstens eine genannte Nachweissonde umfasst, die mit einem elektrochemischen Nachweismittel gekoppelt ist, so dass eine Hybridisierung einer genannten Kennmarke mit einer komplementären Nachweissonde gemessen werden kann,
wobei der genannte Universaldetektor eine Anordnung von Nachweissonden umfasst, die mit Nachweismitteln gekoppelt sind, wobei die genannte Anordnung an einem Substrat angebrachte Elektroden umfasst;
wobei das Messen der Hybridisierung einer genannten Kennmarke mit einer genannten komplementären Nachweissonde zeigt, dass das genannte Ziel, das der genannten Kennmarke entspricht, in der genannten untersuchten Probe vorliegt.

36. Zusammensetzung nach Anspruch 35, wobei das genannte wenigstens eine markierte Molekül die Kennmarke für ein Ziel umfasst und ferner eine Kopie oder ein Komplement des genannten Ziels umfasst.

37. Zusammensetzung nach Anspruch 35, wobei das genannte wenigstens eine markierte Molekül das Kennmarkenmolekül für ein Ziel umfasst und keine Kopie und kein Komplement des genannten Ziels enthält.

38. Zusammensetzung nach Anspruch 35, wobei die genannten Elektroden aus Gold oder Kohlenstoff sind.

39. Zusammensetzung nach Anspruch 36, wobei die genannten Elektroden aus Gold sind.

40. Zusammensetzung nach Anspruch 35, wobei die genannten Elektroden mit Protein beschichtet sind, das durch Oligonukleotide gebunden werden kann, die mit einem Anteil derivatisiert sind, der das genannte Protein bildet, mit dem die genannte Elektrode beschichtet ist.

41. Zusammensetzung nach Anspruch 35, wobei die genannten Elektroden mit Avidin beschichtet sind, so dass die genannten Elektroden durch Biotin-etikettierte Oligonukleotide gebunden werden können.

42. Zusammensetzung nach Anspruch 35, wobei der genannte Satz von minimal querhybridisierenden Marken und Sonden so ausgewählt ist, dass alle Sonden/Marke-Duplexe dieselbe oder eine ähnliche Schmelztemperatur und Stapelenergie haben.

## Revendications

1. Procédé pour détecter une séquence nucléotidique cible dans un échantillon, ledit procédé comprenant les étapes consistant à :
a) générer au moins une molécule marquée comprenant au moins un marqueur d'identification sélectionné comme identifiant pour ladite séquence nucléotidique cible, où ledit marqueur d'identification est généré uniquement lorsque ladite séquence nucléotidique cible correspondant audit marqueur d'identification est présente dans ledit échantillon ;
b) incuber ladite au moins une molécule marquée avec un détecteur universel ayant au moins une sonde de détection complémentaire audit marqueur d'identification, ledit détecteur universel comprenant une matrice de sondes de détection couplées à des moyens de détection électrochimique, ladite matrice comprenant des électrodes fixées à un substrat ; et
c) détecter l'hybridation de tout dit marqueur d'identification à toute dite sonde de détection complémentaire audit marqueur d'identification, où ladite hybridation de tout dit marqueur d'identification à toute dite sonde de détection complémentaire audit marqueur d'identification indique que ladite séquence nucléotidique cible correspondant audit marqueur d'identification est présente dans ledit échantillon.

2. Procédé de la Revendication 1, où ladite au moins une molécule marquée comprend en outre une copie ou un complément de ladite séquence nucléotidique cible.

3. Procédé de la Revendication 2, où lesdites sondes de détection couplées à un moyen de détection sont fixées à une surface, film ou particule.

4. Procédé de la Revendication 6, où lesdites sondes de détection sont fixées à ladite surface, film ou particule par des liaisons covalentes, liaisons ioniques, interactions électrostatiques ou adsorption.

5. Procédé de la Revendication 1, où lesdites électrodes sont en or ou en carbone.

6. Procédé de la Revendication 11, où lesdites électrodes sont en or.

7. Procédé de la Revendication 1, où l'étape de détection d'hybridation consiste à mesurer l'hybridation de tout dit marqueur d'identification à toute dite sonde de détection complémentaire audit marqueur d'identification en utilisant un complexe métallique de transition capable d'oxyder au moins une base oxydable dans une réaction d'oxydoréduction dans des conditions qui engendrent une réaction d'oxydoréduction entre ledit complexe métallique de transition et ladite base oxydable, où ladite sonde de détection et/ou ledit marqueur d'identification comprennent au moins une base oxydable.

8. Procédé de la Revendication 7, où ladite étape de détection d'hybridation comprend une ampérométrie au ruthénium.

9. Procédé de la Revendication 1, où lesdites électrodes sont revêtues d'une protéine qui peut être liée par des oligonucléotides dérivés avec une fraction qui lie ladite protéine qui revêt ladite électrode.

10. Procédé de la Revendication 9, où lesdites électrodes sont revêtues d'avidine de telle sorte que lesdites électrodes puissent être liées par des oligonucléotides marqués avec de la biotine.

11. Procédé d'une quelconque revendication précédente, où ladite étape de génération d'au moins une molécule marquée comprenant au moins un marqueur d'identification sélectionné comme identifiant pour ladite séquence nucléotidique cible consiste à amplifier un modèle présent dans ledit échantillon, où ledit modèle comprend ladite séquence nucléotidique cible ou complément de celle-ci.

12. Procédé d'une quelconque revendication précédente pour détecter une pluralité de séquences nucléotidiques cibles dans un échantillon, où chaque séquence nucléotidique cible dans ladite pluralité de séquences nucléotidiques cibles a un marqueur d'identification distinct, de telle sorte que l'hybridation de chaque dit marqueur d'identification distinct à une sonde de détection complémentaire indique la présence de la séquence nucléotidique cible correspondante.

13. Procédé d'une quelconque revendication précédente, où ladite au moins une molécule marquée comprend une séquence nucléotidique exogène non décelée dans ledit marqueur d'identification ou ladite séquence nucléotidique cible.

14. Procédé de la Revendication 13, où ladite séquence nucléotidique exogène est une séquence impliquée dans la découpe de molécules marquées et ledit procédé comprend en outre la découpe de ladite au moins une molécule marquée pour générer au moins une plus petite molécule marquée.

15. Procédé de la Revendication 11, où ladite amplification dudit modèle comprend l'amplification par cercle roulant (RC) comprenant les étapes consistant à :
a) fournir une sonde à cercle roulant (RC) comprenant une séquence complémentaire audit modèle comprenant une séquence nucléotidique cible ou un complément de celle-ci, et comprenant en outre une séquence complémentaire audit marqueur d'identification sélectionné comme identifiant pour ladite séquence nucléotidique cible ;
b) incuber ladite sonde RC avec ledit modèle dans des conditions telles que ladite sonde RC s'hybridera à la séquence complémentaire sur ledit modèle;
c) fournir une enzyme polymérase dans des conditions telles que ladite polymérase réplique ladite sonde RC, générant au moins un produit d'amplification comprenant une molécule marquée ayant des copies de répétition dudit marqueur d'identification et de ladite séquence nucléotidique cible ou complément de celle-ci ;
d) incuber ledit produit d'amplification avec un détecteur universel comprenant des sondes de détection couplées à un moyen de détection, lesdites sondes de détection comprenant au moins une sonde de détection complémentaire audit marqueur d'identification ; et
e) détecter l'hybridation de tout dit marqueur d'identification dans ledit produit d'amplification à toute dite sonde de détection complémentaire audit marqueur d'identification ;
où l'hybridation de tout dit marqueur d'identification à toute dite sonde de détection complémentaire indique la présence de la séquence nucléotidique cible correspondante dans l'échantillon analysé.

16. Procédé de la Revendication 15, où ladite sonde RC fournie à l'étape a) est une sonde RC circulaire.

17. Procédé de la Revendication 15, où ladite sonde RC fournie à l'étape a) est linéaire, de telle sorte que l'étape b) comprend en outre l'incubation de ladite sonde RC avec ledit modèle dans des conditions telles que l'extrémité 3' de ladite sonde RC et l'extrémité 5' de ladite sonde RC s'hybrideront de manière adjacente à la séquence complémentaire contigüe sur ledit modèle, et ladite extrémité 3' et ladite extrémité 5' sont ligaturées pour Former une sonde cadenas RC circulaire.

18. Procédé de la Revendication 11 ou d'une quelconque des Revendications 15 à 17, où ledit modèle est de l'ADN ou ARN.

19. Procédé d'une quelconque des Revendications 15 à 17, où ledit au moins un produit d'amplification comprend en outre une séquence exogène non décelée dans ledit marqueur d'identification ou ledit modèle comprenant une séquence nucléotidique cible ou un complément de celle-ci.

20. Procédé de la Revendication 19, où ladite séquence exogène est impliquée dans une liaison d'amorce audit produit d'amplification, formant des promoteurs de polymérase sur ledit produit d'amplification, ou découpant ledit produit d'amplification.

21. Procédé de la Revendication 20, où ladite séquence exogène impliquée dans la découpe dudit produit d'amplification comprend des séquences auto-complémentaires qui forment des structures en épingle à cheveux.

22. Procédé de la Revendication 21, où lesdites séquences auto-complémentaires qui forment des structures en épingle à cheveux comprennent au moins un site de reconnaissance d'enzyme de restriction pour une enzyme de restriction impliquée dans ladite découpe.

23. Procédé de la Revendication 20, où ladite au moins une séquence nucléotidique exogène comprend des séquences qui forment au moins un site de reconnaissance d'enzyme de restriction pour une enzyme de restriction impliquée dans ladite découpe lors de l'ajout d'au moins un oligonucléotide auxiliaire.

24. Procédé de la Revendication 11, où ladite amplification dudit modèle comprend une amplification induite par ligase consistant à :
a) fournir au moins une paire d'amorces de ligature comprenant une première amorce de ligature ayant une partie 3' de séquence complémentaire à une partie dudit modèle comprenant ladite séquence nucléotidique cible ou complément de celle-ci, et une seconde amorce de ligature ayant une partie 5' de séquence complémentaire à une partie contigüe dudit modèle comprenant une séquence nucléotidique cible ou un complément de celle-ci, où au moins une amorce de ligature comprend en outre un marqueur d'identification sélectionné comme identifiant pour ladite séquence nucléotidique cible ;
b) incuber ladite au moins une paire d'amorces de ligature avec ledit modèle dans des conditions telles que lesdites première et seconde amorces de ligature s'hybrideront audit modèle, ladite extrémité 3' de ladite première amorce de ligature s'hybridant de manière adjacente à ladite extrémité 5' de ladite seconde amorce de ligature ;
c) ligaturer ladite extrémité 3' de ladite première amorce de ligature à ladite extrémité 5' de ladite seconde amorce de ligature, générant ainsi un produit de ligature comprenant une molécule marquée, où ladite molécule marquée comprend une copie ou un complément de ladite séquence nucléotidique cible et comprend en outre au moins un marqueur d'identification correspondant à ladite séquence nucléotidique cible ;
d) mettre en contact ledit produit de ligature avec un détecteur universel comprenant des sondes de détection couplées à un moyen de détection, lesdites sondes de détection comprenant au moins une sonde de détection complémentaire audit marqueur d'identification ; et
e) détecter l'hybridation de tout dit marqueur d'identification dans ledit produit de ligature à toute dite sonde de détection complémentaire audit marqueur d'identification ;
où l'hybridation de tout dit marqueur d'identification à toute dite sonde de détection complémentaire indique la présence de la séquence nucléotidique cible correspondante dans l'échantillon analysé.

25. Procédé de la Revendication 24, où ledit modèle comprend une séquence nucléotidique cible, de telle sorte que la première amorce de ligature comprend une séquence complémentaire à une partie de ladite séquence nucléotidique cible et ladite seconde amorce de ligature comprend une séquence complémentaire à une partie contigüe de ladite séquence nucléotidique cible, avec comme résultat le fait que ladite première amorce de ligature et ladite seconde amorce de ligature s'hybrident à des parties contigües de modèle et sont ligaturées, générant un produit de ligature comprenant un marqueur d'identification et une séquence complémentaire à ladite séquence nucléotidique cible.

26. Procédé de la Revendication 24, où ledit modèle comprend un complément de séquence nucléotidique cible, de telle sorte que la première amorce de ligature comprend une partie de ladite séquence nucléotidique cible et ladite seconde amorce de ligature comprend une partie contigüe de ladite séquence nucléotidique cible, avec comme résultat le fait que ladite première amorce de ligature et ladite seconde amorce de ligature s'hybrident à des parties contigües de modèle et sont ligaturées, générant un produit de ligature comprenant un marqueur d'identification et ladite séquence nucléotidique cible.

27. Procédé d'une quelconque des Revendications 24 à 26, où ladite ligature de ladite extrémité 3' de ladite première amorce de ligature à ladite extrémité 5' de ladite seconde amorce de ligature est effectuée par des moyens enzymatiques.

28. Procédé de la Revendication 27, où lesdits moyens enzymatiques sont une ligase.

29. Procédé d'une quelconque des Revendications 24 à 26, ou ladite ligature de ladite extrémité 3' de ladite première amorce de ligature à ladite extrémité 5' de ladite seconde amorce de ligature est effectuée par des moyens non enzymatiques.

30. Procédé d'une quelconque des Revendications 24 à 26, où lesdites étapes d'incubation et de ligature sont répétées en utilisant des cycles de température pour l'amplification de ladite séquence nucléotidique cible.

31. Procédé d'une quelconque des Revendications 24 à 26, pour détecter une pluralité de séquences nucléotidiques cibles, consistant à fournir une pluralité de paires d'amorces de ligature où au moins une dite paire d'amorces de ligature de ladite pluralité de paires est complémentaire à chaque séquence nucléotidique cible de ladite pluralité de séquences nucléotidiques cibles, et en outre où au moins une amorce de ligature de chaque dite paire comprend un marqueur d'identification sélectionné pour servir d'identifiant pour ladite séquence nucléotidique cible.

32. Procédé d'une quelconque des Revendications 24 à 26, ou ledit procédé comprend la détection d'au moins une séquence variante de ladite séquence nucléotidique cible.

33. Procédé de la Revendication 32, où ladite au moins une séquence variante est un polymorphisme mononucléotidique (SNP), une variante allélique ou une variante d'épissage.

34. Procédé de la Revendication 33, où ladite au moins une séquence variante est un SNP.

35. Composition pour détecter des cibles dans un échantillon utilisant un essai de marqueur universel comprenant :
a) un ensemble de marqueurs et sondes d'hybridation croisée minimale sélectionné de telle sorte qu'au moins un marqueur servira de marqueur d'identification pour une cible dans un échantillon analysé et chaque dit marqueur d'identification a au moins une sonde de détection complémentaire dans ledit ensemble ;
b) au moins une molécule marquée comprenant au moins un dit marqueur d'identifcation, où ledit marqueur d'identification est généré uniquement lorsque ladite cible correspondant audit marqueur d'identification est présente dans ledit échantillon analysé ; et
c) un détecteur universel comprenant au moins une dite sonde de détection couplée à un moyen de détection électrochimique de telle sorte que l'hybridation de tout dit marqueur d'identification à toute dite sonde de détection complémentaire puisse être mesurée
où ledit détecteur universel comprend une matrice de sondes de détection couplées à des moyens de détection, ladite matrice comprenant des électrodes fixées à un substrat ;
où la mesure de hybridation de tout dit marqueur d'identification à toute dite sonde de détection complémentaire indique que ladite cible correspondant audit marqueur d'identification est présente dans ledit échantillon analysé.

36. Composition de la Revendication 35, où ladite au moins une molécule marquée comprend le marqueur d'identification pour une cible et comprend en outre une copie ou un complément de ladite cible.

37. Composition de la Revendication 35, où ladite au moins une molécule marquée comprend la molécule de marqueur d'identification pour une cible et ne contient pas de copie ou complément de ladite cible.

38. Composition de la Revendication 35, où lesdites électrodes sont en or ou en carbone.

39. Composition de la Revendication 36, où lesdites électrodes sont en or

40. Composition de la Revendication 35, où lesdites électrodes sont revêtues d'une protéine qui peut être liée par des oligonucléotides dérivés avec une fraction qui lie ladite protéine qui revêt ladite électrode.

41. Composition de la Revendication 35, où lesdites électrodes sont revêtues d'avidine de telle sorte que lesdites électrodes puissent être liées par des oligonucléotides marqués avec de la biotine.

42. Composition de la Revendication 35, où ledit ensemble de marqueurs et sondes d'hybridation croisée minimale est sélectionné de telle sorte que tous les duplexes sonde/marqueur aient une température de fusion et énergie accumulée égale ou similaire.


### SEQUENCE LISTING

<110> GeneOhm Sciences, Inc.
   Crothers, Donald M.
   Holmlin, R. Erik
<120> UNIVERSAL TAG ASSAY
<130> GENOM.019VPC
<140> Unknown
   <141> - -
<150> 60/424,656
   <151> 2002-11-06
<150> 10/424,542
   <151> 2003-04-24
<160> 11
<170> FastSEQ for Windows Version 4.0
<210> 1
   <211> 24
   <212> DNA
   <213> Artificial Sequence
<220>
   <223> 5' end of rolling circle probe
<400> 1
   gcacctcaaa gctgttccgt ccca 24
<210> 2
   <211> 13
   <212> DNA
   <213> Artificial Sequence
<220>
   <223> 3' end of rolling circle probe
<400> 2
   caggcacaaa cac 13
<210> 3
   <211> 15
   <212> DNA
   <213> Artificial Sequence
<220>
   <223> 2' tag
<400> 3
   agctactggc aatct 15
<210> 4
   <211> 20
   <212> DNA
   <213> Artificial Sequence
<220>
   <223> T7 promoter
<400> 4
   ccctatagtg agtcgtatta 20
<210> 5
   <211> 6
   <212> DNA
   <213> Artificial Sequence
<220>
   <223> Eco RI site
<400> 5
   gaattc 6
<210> 6
   <211> 3
   <212> DNA
   <213> Artificial Sequence
<220>
   <223> 3 nucleotide
<400> 6
   gat 3
<210> 7
   <211> 21
   <212> DNA
   <213> Artificial Sequence
<220>
   <223> Rolling circle primer
<400> 7
   gataggagtc acttaagatc g 21
<210> 8
   <211> 98
   <212> DNA
   <213> Artificial Sequence
<220>
   <223> Rolling circle probe
<400> 8
   gcacctcaaa gctgttccgt cccagttgac tatcctcagt gaattctagc tactggcaat 60
   ctgatcccta tagtgagtcg tattacaggc acaaacac                         98
<210> 9
   <211> 15
   <212> DNA
   <213> Artificial Sequence
<220>
   <223> Detection probe sequence
<400> 9
   gtacggataa ctacg 15
<210> 10
   <211> 75
   <212> DNA
   <213> rolling circle probe sequence
<400> 10
   gcctgtccag ggatctgctc ttaccctata gtgagtcgta ttacgtagtt atccgtacca 60
   atacctgtat tcctt                                                   75
<210> 11
   <211> 15
   <212> DNA
   <213> Artificial Sequence
<220>
   <223> Signal probe sequence
<400> 11
   cctatagtga gtcgt 15